# EUROPEAN PATENT SPECIFICATION

(11) **EP 3 743 720 B1**
(45) Date of publication and mention of the grant of the patent: **14.08.2024**
(21) Application number: 19743538.1
(22) Date of filing: 24.01.2019
(51) Int. Cl.: G01N 33/48, G01N 33/50, G01N 33/53, G01N 33/558, G01N 33/543

(54) **MULTIPLEX LATERAL FLOW ASSAY FOR DIFFERENTIATING BACTERIAL INFECTIONS FROM VIRAL INFECTIONS**
MULTIPLEX-LATERAL-FLOW-TEST ZUR DIFFERENZIERUNG BAKTERIELLER INFEKTIONEN VON VIRALEN INFEKTIONEN
ANALYSE À ÉCOULEMENT LATÉRAL MULTIPLEX PERMETTANT LA DIFFÉRENCIATION ENTRE INFECTIONS BACTÉRIENNES ET INFECTIONS VIRALES

(30) Priority: 27.01.2018 US 201862622878 P
(43) Date of publication of application: 02.12.2020
(73) Proprietor: Becton, Dickinson and Company, Franklin Lakes, NJ 07417 (US)
(72) Inventor: REN, Huimiao, Franklin Lakes, New Jersey 07417 (US); YANG, Jian, Franklin Lakes, New Jersey 07417 (US); LIU, Guohong, Franklin Lakes, New Jersey 07417 (US)
(74) Representative: Vossius & Partner Patentanwälte Rechtsanwälte mbB
(86) International application number: PCT/US2019/015008
(87) International publication number: WO 2019/147853

(56) References cited:
- WO-A1-2014/070686
- WO-A1-2019/005694
- US-A1- 2006 246 601
- US-A1- 2007 224 701
- US-A1- 2007 224 701
- US-A1- 2007 243 630
- US-A1- 2007 243 630
- US-A1- 2010 068 826
- US-A1- 2014 170 642
- US-A1- 2014 377 879
- US-A1- 2016 153 993
- US-A1- 2016 153 993
- VASHIST SANDEEP KUMAR ET AL: "Bioanalytical advances in assays for C-reactive protein", BIOTECHNOLOGY ADVANCES, ELSEVIER PUBLISHING, BARKING, GB, vol. 34, no. 3, 21 December 2015 (2015-12-21), pages 272 - 290, XP029497782, ISSN: 0734-9750, DOI: 10.1016/J.BIOTECHADV.2015.12.010
- ELIZABETH G. REY ET AL: "Mitigating the Hook Effect in Lateral Flow Sandwich Immunoassays Using Real-Time Reaction Kinetics", ANALYTICAL CHEMISTRY, vol. 89, no. 9, 2 May 2017 (2017-05-02), US, pages 5095 - 5100, XP055566188, ISSN: 0003-2700, DOI: 10.1021/acs.analchem.7b00638

## Description

### CROSS REFERENCE TO RELATED APPLICATION

### FIELD

The present disclosure relates in general to lateral flow assay devices, test systems, and methods. More particularly, the present disclosure relates to lateral flow assay devices to determine the presence and concentration of a plurality of analytes in a sample, including when one or more analytes of interest are present at high concentrations and one or more analytes of interest are present at low concentrations. Precise concentration of each of the plurality of analytes can be determined when a single sample is applied to a single lateral flow assay in a single application, including when a first analyte of interest is present in the single sample at one-millionth the concentration of a second analyte of interest in the single sample.

### BACKGROUND

Immunoassay systems, including lateral flow assays described herein provide reliable, inexpensive, portable, rapid, and simple diagnostic tests. Lateral flow assays can quickly and accurately detect the presence or absence of, and in some cases quantify, an analyte of interest in a sample. Advantageously, lateral flow assays can be minimally invasive and used as point-of-care testing systems. Lateral flow assays have been developed to detect a wide variety of medical or environmental analytes. In a sandwich format lateral flow assay, a labeled antibody against an analyte of interest is deposited on a test strip in or near a sample receiving zone. The labeled antibody may include, for example, a detector molecule or "label" bound to the antibody. When the sample is applied to the test strip, analyte present in the sample is bound by the labeled antibody, which flows along the test strip to a capture zone, where an immobilized antibody against the analyte binds the labeled antibody-analyte complex. The antibody immobilized on the capture line may be different than the labeled antibody deposited in or near the sample receiving zone. The captured complex is detected, and the presence of analyte is determined. In the absence of analyte, the labeled antibody flows along the test strip but passes by the capture zone. The lack of signal at the capture zone indicates the absence of analyte. Multiplex assays can be developed to detect more than one analyte of interest present in a single sample applied to a lateral flow assay, but such assays suffer from many disadvantages, including cross reactivity between antibodies and analyes of interest; the inability to detect, using one optical reader, multiple analytes of interest applied to a single test strip during a single test event; and the inability to detect analytes of interest that are present in a single sample at significantly different concentrations. Typically a sample with a high concentration analyte must first be diluted in order to test for the presence or concentration of the high concentration analyte. Such dilution further lowers the concentration of any analytes of interest that are present in the sample at low concentration, rendering the low-concentration analytes undetectable. To date, multiplex lateral flow assays have not been suitable to determine the quantity and presence of a plurality of analytes in a sample, where one or more analytes are present in high concentration and one or more analytes are present at low concentration. Document WO2014/070686 discloses a multiplex lateral flow assay for detecting CRP at high concentration and IL-6 in one single assay.

### SUMMARY

It is therefore an aspect of this disclosure to provide improved lateral flow assays for detecting the presence and the concentration of a plurality of analytes of interest in a sample, when a first analyte is present in the sample at a high concentration and a second, different analyte is present in the sample at a low concentration, including but not limited to a concentration that is one-millionth the high concentration.

In one embodiment of the present disclosure, a method of detecting a first analyte of interest and a second analyte of interest present in a sample at different concentrations is provided. The method includes providing a lateral flow assay including a first complex coupled to a flow path of the lateral flow assay, the first complex including a label, an antibody or a fragment thereof that specifically binds the first analyte, and the first analyte. The lateral flow assay also includes a labeled second antibody or fragment thereof coupled to the flow path and configured to specifically bind the second analyte. The lateral flow assay further includes a first capture zone downstream of the first complex, the first capture zone including a first immobilized capture agent specific to the first analyte. The lateral flow assay also includes a second capture zone downstream of the labeled second antibody or fragment thereof and including a second immobilized capture agent specific to the second analyte. The method also includes applying the sample to the first complex and the labeled second antibody or fragment thereof; and binding the second analyte to the labeled second antibody or fragment thereof to form a second complex. The method further includes flowing the fluid sample and the first complex to the first capture zone, where the first analyte in the fluid sample and the first complex compete to bind to the first immobilized capture agent in the first capture zone; and flowing the second complex in the flow path to the second capture zone and binding the second complex to the second immobilized capture agent in the second capture zone. The method also includes detecting a first signal from the first complex bound to the first immobilized capture agent in the first capture zone and a second signal from the second complex bound to the second immobilized capture agent in the second capture zone.

In another embodiment of the present disclosure, a lateral flow assay configured to detect a first analyte of interest and a second analyte of interest present in a fluid sample at different concentrations is provided. The lateral flow assay includes a first complex coupled to a flow path of the lateral flow assay, the first complex including a label, an antibody or a fragment thereof that specifically binds the first analyte, and the first analyte; a labeled second antibody or fragment thereof coupled to the flow path and configured to specifically bind the second analyte; a first capture zone downstream of the first complex, the first capture zone including a first immobilized capture agent specific to the first analyte; and a second capture zone downstream of the labeled second antibody or fragment thereof and including a second immobilized capture agent specific to the second analyte.

In still another embodiment of the present disclosure, not part of the claimed invention, an assay test strip is provided. The assay test strip includes a flow path configured to receive a fluid sample; a sample receiving zone coupled to the flow path; and a detection zone coupled to the flow path downstream of the sample receiving zone. The detection zone includes a first capture zone, a second capture zone, and a third capture zone. The first capture zone includes a first immobilized capture agent specific to a first analyte of interest, the second capture zone includes a second immobilized capture agent specific to a second analyte of interest, and the third capture zone includes a third immobilized capture agent specific to a third analyte of interest. The assay test strip also includes a first complex coupled to the flow path in a first phase and configured to flow in the flow path to the detection zone in the presence of the fluid sample in a second phase. The first complex includes a label, a first antibody or a fragment thereof that specifically binds the first analyte of interest, and the first analyte of interest. The assay test strip further includes a labeled second antibody or fragment thereof that specifically binds the second analyte of interest, the labeled second antibody or fragment thereof coupled to the flow path in the first phase and configured to flow in the flow path to the detection zone in the presence of the fluid sample in the second phase. The assay test strip also includes a labeled third antibody or fragment thereof that specifically binds the third analyte of interest, the labeled third antibody or fragment thereof coupled to the flow path in the first phase and configured to flow in the flow path to the detection zone in the presence of the fluid sample in the second phase.

In still a further embodiment of the present disclosure, not part of the claimed invention, a diagnostic test system is provided. The diagnostic test system includes an assay test strip as described above; a reader including a light source and a detector, and a data analyzer.

In another embodiment of the present disclosure, not part of the claimed invention, a method of determining a presence or a concentration of each of a plurality of analytes of interest in a fluid sample is provided. The method includes applying the fluid sample to an assay test strip described above when the first complex, the labeled second antibody or fragment thereof, and the labeled third antibody or fragment thereof are each coupled to the flow path in the first phase. The method also includes binding the second analyte, if present in the fluid sample, to the labeled second antibody or fragment thereof, thereby forming a second complex; binding the third analyte, if present in the fluid sample, to the labeled third antibody or fragment thereof, thereby forming a third complex; uncoupling the first complex, the second complex, if formed, and the third complex, if formed, from the flow path; flowing the fluid sample to the detection zone in the second phase; binding the first complex to the first immobilized capture agent in the first capture zone, binding the second complex, if formed, to the second immobilized capture agent in the second capture zone, and binding the third complex, if formed, to the third immobilized capture agent in the third capture zone; detecting a first signal from the first complex bound to the first immobilized capture agent in the first capture zone; if the second complex is formed, detecting a second signal from the second complex bound to the second immobilized capture agent in the second capture zone; and if the third complex is formed, detecting a third signal from the third complex bound to the third immobilized capture agent in the third capture zone.

### BRIEF DESCRIPTION OF THE DRAWINGS

Figures 1A and 1B illustrate an example lateral flow assay according to the present disclosure before and after a fluid sample is applied at a sample receiving zone, where the fluid sample includes a first analyte of interest, a second analyte of interest, and a third analyte of interest.
Figures 2A and 2B illustrate an example lateral flow assay according to the present disclosure before and after a fluid sample is applied at a sample receiving zone, where the fluid sample does not include any analyte of interest.
Figures 3A and 3B illustrate an example lateral flow assay according to the present disclosure before and after a fluid sample is applied at a sample receiving zone, where the fluid sample includes a first analyte of interest but does not include a second or a third analyte of interest.
Figures 4A and 4B illustrate an example lateral flow assay according to the present disclosure before and after a fluid sample is applied at a sample receiving zone, where the fluid sample includes a second analyte of interest but does not include a first or a third analyte of interest.
Figures 5A and 5B illustrate an example lateral flow assay according to the present disclosure before and after a fluid sample is applied at a sample receiving zone, where the fluid sample includes a third analyte of interest but does not include a first or a second analyte of interest.
Figures 6A and 6B illustrate an example lateral flow assay according to the present disclosure before and after a fluid sample is applied at a sample receiving zone, where the fluid sample includes a first analyte of interest and a third analyte of interest, but not a second analyte of interest.
Figure 7A illustrates an example dose response curve for an example lateral flow assay such as that illustrated in Figures 3A and 3B, where the fluid sample includes only C-reactive protein (CRP) in a concentration of up to 150 µg/mL, and where the fluid sample does not include any additional analytes of interest.
Figure 7B illustrates an example dose response curve for an example lateral flow assay such as that illustrated in Figures 4A and 4B, where the fluid sample includes only Analyte 2 in a concentration of up to 1000 pg/mL, and where the fluid sample does not include any additional analytes of interest, such as Analyte 3 or CRP.
Figure 7C illustrates an example dose response curve for an example lateral flow assay such as that illustrated in Figures 5A and 5B, where the fluid sample includes only Analyte 3 in a concentration of up to 500 pg/mL, and where the fluid sample does not include any additional analytes of interest, such as Analyte 2 or CRP.
Figure 8 illustrates example lateral flow assay devices according to the present disclosure including a sample receiving zone and a detection zone. The detection zone may include an indication of the presence and/or concentration of a plurality of analytes in a fluid sample, such as but not limited to CRP, PCT, and Mx1, including when one or more analytes of interest are present at high concentration and when one or more analytes of interest are present at low concentration.

### DETAILED DESCRIPTION

Devices, systems and methods described herein precisely determine the quantity or presence of a plurality of analytes of interest in a sample. Lateral flow devices, test systems, and methods according to the present disclosure precisely determine the presence or quantity of a plurality of analytes of interest in situations where one or more analytes of interest are present in the sample at an elevated or high concentration and one or more analytes of interest are present in the sample at a low concentration. Advantageously, lateral flow devices, test systems, and methods described herein determine the presence or quantity of analytes of interest present in a single sample at significantly different concentrations after applying the single sample to one lateral flow assay, such as a single test strip, in a single test event. Lateral flow assays described herein are thus capable of detecting a plurality of analytes simultaneously, in a single sample, even when analytes are present in significantly different concentration ranges.

Lateral flow assays described herein can use a combination of binding assays on a single test strip, including an assay for detecting one or more analytes present at a high concentration in combination with an assay for detecting one or more analytes present at a low concentration. The single test strip of lateral flow assays described herein can include a detection zone having a separate capture zone specific for each analyte of interest. For example, a sample may include three analytes of interest: a first analyte of interest, a second analyte of interest, and a third analyte of interest. The detection zone of the lateral flow assay would thus include three capture zones: a first capture zone specific to the first analyte of interest, a second capture zone specific to the second analyte of interest, and a third capture zone specific to the third analyte of interest.

In this non-limiting example, the first analyte of interest may be present in the sample at high concentrations, such as but not limited to a range of 1-999 µg/ml. The lateral flow assay described herein can generate a signal of maximum intensity at the first capture zone when the concentration of the first analyte of interest in the sample is zero. Increasing concentrations of the first analyte of interest decrease the signal from the maximum intensity signal to a reduced intensity signal, which can be correlated to a concentration for the first analyte of interest. In this example, the second analyte of interest and the third analyte of interest may be present in the sample at low concentrations, such as but not limited to a range of 1-999 pg/ml. The lateral flow assay described herein can generate a signal intensity at the second capture zone and the third capture zone with increasing signal intensity correlated to increasing concentration of the second analyte of interest and the third analyte of interest, respectively. Thus, the lateral flow assay according to the present disclosure can detect high concentration and low concentration analytes using a single assay, such as a single test strip.

Lateral flow assays according to the present disclosure can measure the presence and concentration of multiple analytes of interest present at significantly different concentrations in a single, undiluted sample that is applied, in a single test event, to a single lateral flow assay. The ability to measure the presence and concentration of multiple analytes of interest at very different concentrations (including concentrations six orders of magnitude different, or on the order of one million times different) without diluting the sample offers significant advantages. For example, embodiments of the lateral flow assays described herein can measure analytes present in whole blood, venous blood, capillary blood, serum, and plasma samples that have not been diluted or pre-processed prior to application to the lateral flow assay, such as a single lateral flow assay test strip.

Advantageously, implementations of the lateral flow assay can simultaneously detect low concentration analytes present in the same sample as high concentration analytes, even when the high concentration analyte has a large dynamic range (including but not limited to CRP, which may be present in a sample across a large dynamic range). In addition, the ability to simultaneously and precisely detect the concentration of a plurality of analytes of interest that are present in a single sample at significantly different concentrations (on the order of one millionth the concentration) has significant diagnostic benefits. In one non-limiting example of the lateral flow assay of the present disclosure, measurements of optical signals from a single test strip can be correlated to the presence or absence of a viral infection, a bacterial infection, or no infection in a patient.

Signals generated by assays according to the present disclosure are described herein in the context of an optical signal generated by reflectance-type labels (such as but not limited to gold nanoparticle labels). Although embodiments of the present disclosure are described herein by reference to an "optical" signal, it will be understood that assays described herein can use any appropriate material for a label in order to generate a detectable signal, including but not limited to fluorescence-type latex bead labels that generate fluorescence signals and magnetic nanoparticle labels that generate signals indicating a change in magnetic fields associated with the assay.

According to the present disclosure, a lateral flow assay device includes labeled antibodies designed for detecting high concentration analyte in a sample in combination with labeled antibodies designed for detecting low concentration analyte in the same sample. For example, a sample may include a first analyte of interest at high concentration, a second analyte of interest at low concentration, and a third analyte of interest at low concentration. To detect the first analyte of interest at high concentration, a first complex is initially integrated onto a surface, for example onto a conjugate pad, of a lateral flow assay test strip at a receiving zone or label zone. The first complex includes a label, a first antibody that specifically binds the first analyte of interest, and the first analyte of interest. The first complex becomes unbound from the label zone upon application of a fluid sample to the test strip, and travels to a detection zone of the test strip with the fluid sample, which may include a first analyte of interest. The detection zone includes a capture zone specific for each analyte of interest, and thus includes a first capture zone for capturing the first analyte of interest, a second capture zone for capturing the second analyte of interest, and a third capture zone for detecting a third analyte of interest. The first complex and the first analyte of interest in the sample (when present) bind to a first capture agent in the first capture zone. The first capture agent binds solely to the first complex when there is no first analyte of interest present in the sample, which would otherwise compete with the first complex. Thus, a first signal having maximum intensity is generated at the first capture zone when no first analyte of interest is present in the sample. When first analyte of interest is present in the sample in low concentrations, the first complex competes with a relatively low amount of first analyte to bind to first capture agent, resulting in a first signal that is the same as or substantially equivalent to (within a limited range of variance from) the first signal having maximum intensity. When first analyte of interest is present in the sample in high concentrations, the first complex competes with a relatively high amount of first analyte to bind to first capture agent, resulting in a first signal that is less than the signal having maximum intensity.

To detect the second analyte of interest (present in the sample at low concentration in this non-limiting example), a labeled second antibody that specifically binds the second analyte of interest is initially integrated onto a surface, for example onto the conjugate pad, of the lateral flow assay test strip at the receiving zone or label zone. The labeled second antibody becomes unbound from the label zone upon application of the fluid sample to the test strip, and binds to the second analyte of interest to form a second complex. The second complex travels to the detection zone of the test strip with the fluid sample. The second complex binds to a second capture agent that is specific to the second analyte of interest in the second capture zone. As a result, a second signal is generated at the second capture zone when the second analyte of interest is present in the sample. When second analyte of interest is absent from the sample (or present below the detectable level), no second complex forms (or less than a detectable amount of second complex forms), and thus no second complex is captured at the second capture zone (or no detectable amount of second complex is captured at the second capture zone). In this situation, the labeled second antibody travels to the detection zone of the test strip with the fluid sample, but it does not bind to the second capture agent in the second capture zone. As a result, no second signal is detected at the second capture zone. Signal intensity of the second signal correlates with concentration of the second analyte of interest, wherein increased signal intensity is correlated to increased concentration of the second analyte of interest in the sample.

Similarly, to detect the third analyte of interest (present in the sample at low concentration in this non-limiting example), a labeled third antibody that specifically binds the third analyte of interest is initially integrated onto a surface, for example onto the conjugate pad, of the lateral flow assay test strip at the receiving zone or label zone. The labeled third antibody becomes unbound from the label zone upon application of the fluid sample to the test strip, and binds to third analyte of interest to form a third complex. The third complex travels to the detection zone of the test strip with the fluid sample. The third complex binds to a third capture agent that is specific to the third analyte of interest in the third capture zone. As a result, a third signal is generated at the third capture zone when the third analyte of interest is present in the sample. When third analyte of interest is absent from the sample (or present below the detectable level), no third complex forms (or no detectable amount of third complex forms), and thus no third complex is captured at the third capture zone (or no detectable amount of third complex is captured at the third capture zone). In this situation, the labeled third antibody travels to the detection zone of the test strip with the fluid sample, but it does not bind to the third capture agent in the third capture zone. As a result, no third signal is detected at the third capture zone. Signal intensity of the third signal correlates with concentration of the third analyte of interest, wherein increased signal intensity is correlated to increased concentration of the third analyte of interest in the sample.

The description above is intended to be illustrative of a circumstance wherein a fluid sample may include a first analyte of interest present at high concentration, a second analyte of interest present at a low concentration, and a third analyte of interest present at a low concentration. One of skill in the art will recognize that the example is intended to be exemplary, and that various modifications and variations may be employed on the lateral flow assays described herein. For example, a fluid sample may include only two analytes of interest, wherein a first analyte is present at high concentration and wherein a second analyte is present at low concentration. Alternatively, a fluid sample may include three analytes of interest, wherein a first analyte of interest is present at high concentration, a second analyte of interest is present at high concentration, and a third analyte of interest is present at low concentration. Furthermore, a fluid sample may include more than three (such as four, five, six, seven, eight, nine, or ten) analytes of interest, with various iterations for a number of analytes at high concentration and a number of analytes present at low concentration. In each of the various iterations, the lateral flow assay is designed as described above to detect simultaneously and on a single lateral flow assay device both the quantity and presence of high concentration analyte and the quantity and presence of low concentration analyte.

One of skill in the art will also recognize that high concentration and low concentration are relative terms, and that the non-limiting implementations below are intended to be illustrate, not limit, the present disclosure. In some non-limiting implementations described below, a first "low concentration" analyte is present in a sample at one millionth the concentration of a second, different "high concentration" analyte present in the same sample. The lateral flow assays according to the present disclosure can measure the presence and concentration of analytes that are present at concentrations in different orders of magnitude, including but not limited to a first analyte of interest that is present at one order of magnitude, two orders of magnitude, three orders of magnitude, four orders of magnitude, five orders of magnitude, six orders of magnitude, seven orders of magnitude, eight orders of magnitude, nine orders of magnitude, and ten orders of magnitude greater than the concentration of a second, different analyte of interest.

Without being bound to any particular theory, the operation of a first complex (which includes a label, a first antibody that specifically binds a first analyte of interest, and the first analyte of interest) together with a second labeled antibody that specifically binds a second analyte of interest, both integrated in the label zone of a single lateral flow assay, will now be described for simultaneous detection and quantification of high concentration analyte and low concentration analyte. Without being bound to any particular theory, the first complex is used to mask the portion of a conventional sandwich-type lateral flow assay dose response curve where signals are increasing (when first analyte concentrations are low), thereby generating a first dose response curve at a first capture zone that starts at a maximum intensity signal at zero concentration of first analyte of interest, and then either remains relatively constant (first analyte at low concentrations) or decreases (first analyte at high concentrations). The second (or additional) labeled antibody that specifically binds the second analyte of interest generates a second dose response curve at a second capture zone that generates an increasing signal intensity with increasing concentration of second analyte. Lateral flow assays of the present disclosure solve drawbacks associated with measuring a plurality of analytes of interest in a sample, particularly where one or more analytes of interest are present at high concentration and one or more analytes of interest are present at low concentration.

In some circumstances, for example, a fluid sample may contain a plurality of analytes of interest, wherein one or more of the analytes of interest are present at high concentration, and one or more of the analytes of interest are present at low concentration. In particular, the one or more analytes of interest may be present in the sample in an amount millions of times greater than the amount of the one or more analytes of interest present at low concentration. Previously, to address this issue, two or more separate tests were required to detect analytes present in a fluid sample at significantly different concentrations. For example, to detect an analyte at high concentration, a sample may be subjected to dilution in order to reduce the high concentration of analyte in the sample to a testable concentration. Dilution of the sample requires additional physical steps of dilution the sample. In addition, dilution also requires additional steps in calculating quantity of an analyte, resulting in more complex algorithms, which may affect the accuracy of the measured quantity of the analyte in the sample. Further, dilution of the sample eliminates the ability to detect analytes present in low concentration because the diluted sample results in a concentration of the analyte present in low concentration below a detectable range. Accordingly, a single sample having analytes at both high and low concentration may be diluted to determine the concentration of the high concentration analyte, but this same sample is not suitable to determine the concentration of the low concentration analyte in conventional multiplex assays.

For detecting low concentration analyte, a sandwich-type lateral flow assay may be used. Conventional sandwich-type lateral flow assays are unsuitable for, and in some cases incapable of, accurately determining a quantity of high concentration analyte. Thus, detection of both high concentration analyte and low concentration analyte present in a single sample previously required application of the sample to multiple detection assays, each assay specifically designed to detect the presence of a particular analyte of interest within the particular dynamic range of that analyte of interest.

In contrast, the lateral flow assay described herein is capable of determining the presence and/or quantity of a plurality of analytes in a fluid sample in a single test (such as a single application of the fluid sample to a single lateral flow assay test strip), wherein one or more of the analytes of interest are present in the fluid sample at high concentration, and one or more analytes of interest are present in the fluid sample at low concentration.

The lateral flow assays described herein include further advantageous features. For example, signals that are generated when a first analyte is at high concentration are readily detectable (for example, they have an intensity within a range of optical signals which conventional readers can typically discern and are well spaced apart), they do not overlap on the dose response curve with signals generated at zero or low concentrations of first analyte, and they can be used to calculate a highly-accurate concentration reading at high and even very high concentrations. In some advantageous implementations, the intensity level of signals generated when a first analyte is present at high concentration do not overlap with the intensity level of signals generated when the first analyte is present at low concentration.

Embodiments of the lateral flow assay described herein are particularly advantageous in diagnostic tests for a plurality of analytes of interest, wherein the relative concentrations of the plurality of interest are indicative of a disease state. When one analyte of interest is present at concentrations above a normal or healthy state, but other analytes of interest are unchanged compared to a normal or healthy state, the diagnosis of the specific disease state may be confidently determined.

Examples of analytes that can be detected and measured by the lateral flow assay devices, test systems, and methods of the present disclosure include the following proteins, without limitation: CRP, PCT, MX1, IP-10, and TRAIL. Implementations of the present disclosure can measure either the soluble and/or the membrane form of the TRAIL protein. In one embodiment, only the soluble form of TRAIL is measured.

Various aspects of the devices, test systems, and methods are described more fully hereinafter with reference to the accompanying drawings. The disclosure may, however, be embodied in many different forms. Based on the teachings herein one skilled in the art should appreciate that the scope of the disclosure is intended to cover any aspect of the devices, test systems, and methods disclosed herein, whether implemented independently of or combined with any other aspect of the present disclosure. For example, a device may be implemented or a method may be practiced using any number of the aspects set forth herein.

Although particular aspects are described herein, many variations and permutations of these aspects fall within the scope of the disclosure. Although some benefits and advantages are mentioned, the scope of the disclosure is not intended to be limited to particular benefits, uses, or objectives. Rather, aspects of the disclosure are intended to be broadly applicable to different detection technologies and device configurations some of which are illustrated by way of example in the figures and in the following description. The detailed description and drawings are merely illustrative of the disclosure rather than limiting, the scope of the disclosure being defined by the appended claims.

Lateral flow devices described herein are analytical devices used in lateral flow chromatography. Lateral flow assays are assays that can be performed on lateral flow devices described herein. Lateral flow devices may be implemented on a test strip but other forms may be suitable. In the test strip format, a test fluid sample, suspected of containing an analyte, flows (for example by capillary action) through the strip. The strip may be made of bibulous materials such as paper, nitrocellulose, and cellulose. The fluid sample is received at a sample reservoir. The fluid sample can flow along the strip to a capture zone in which the analyte (if present) interacts with a capture agent to indicate a presence, absence, and/or quantity of the analyte. The capture agent can include antibody immobilized in the capture zone.

Lateral flow assays can be performed in a sandwich format. Sandwich and assays described herein will be described in the context of reflective-type labels (such as gold nanoparticle labels) generating an optical signal, but it will be understood that assays may include latex bead labels configured to generate fluorescence signals, magnetic nanoparticle labels configured to generate magnetic signals, or any other label configured to generate a detectable signal. Sandwich-type lateral flow assays include a labeled antibody deposited at a sample reservoir on a solid substrate. After sample is applied to the sample reservoir, the labeled antibody dissolves in the sample, whereupon the antibody recognizes and binds a first epitope on the analyte in the sample, forming a label-antibody-analyte complex. This complex flows along the liquid front from the sample reservoir through the solid substrate to a capture zone (sometimes referred to as a "test line"), where immobilized antibodies (sometimes referred to as "capture agent") are located. In some cases where the analyte is a multimer or contains multiple identical epitopes on the same monomer, the labeled antibody deposited at the sample reservoir can be the same as the antibody immobilized in the capture zone. The immobilized antibody recognizes and binds an epitope on the analyte, thereby capturing label-antibody-analyte complex at the capture zone. The presence of labeled antibody at the capture zone provides a detectable optical signal at the capture zone. In one non-limiting example, gold nanoparticles are used to label the antibodies because they are relatively inexpensive, stable, and provide easily observable color indications based on the surface plasmon resonance properties of gold nanoparticles. In some cases, this signal provides qualitative information, such as whether or not the analyte is present in the sample. In some cases, this signal provides quantitative information, such as a measurement of the quantity of analyte in the sample.

Lateral flow assays can provide qualitative information, such as information on the absence or presence of the analyte of interest in the sample. For example, detection of any measurable optical signal at the capture zone can indicate that the analyte of interest is present in the sample (in some unknown quantity). The absence of any measurable optical signal at the capture zone can indicate that the analyte of interest is not present in the sample or below the detection limit. For example, if the sample did not contain any analyte of interest, the sample would still solubilize the labeled agent and the labeled agent would still flow to the capture zone. The labeled agent would not bind to the capture agent at the capture zone, however. It would instead flow through the capture zone, through a control line (if present), and, in some cases, to an optional absorbing zone. Some labeled agent would bind to the control agent deposited on the control line and emit a detectable optical signal. In these circumstances, the absence of a measureable optical signal emanating from the capture zone is an indication that the analyte of interest is not present in the sample, and the presence of a measureable optical signal emanating from the control line is an indication that the sample traveled from the sample receiving zone, through the capture zone, and to the capture line as intended during normal operation of the lateral flow assay.

Some lateral flow devices can provide quantitative information, such as a measurement of the quantity of analyte of interest in the sample. In particular, lateral flow assays can provide reliable quantification of analyte when the analyte is present in low concentration. The quantitative measurement obtained from the lateral flow device may be a concentration of the analyte that is present in a given volume of sample, obtained using a dose response curve that correlates the intensity of a signal detected at the capture zone with the concentration of analyte in the sample. Example signals include optical signals, fluorescence signals, and magnetic signals. For the sandwich-type lateral flow assay, if the sample does not contain any analyte of interest, the concentration of analyte in the sample is zero and no analyte binds to the labeled agent to form a label-antibody-analyte complex. In this situation, there are no complexes that flow to the capture zone and bind to the capture antibody. Thus, no detectable optical signal is observed at the capture zone and the signal magnitude is zero.

A signal is detected as the concentration of analyte in the sample increases with increased analyte concentration in the sample. This takes place because as the analyte concentration increases, the formation of label-antibody-analyte complex increases. Capture agent immobilized at the capture zone binds the increasing number of complexes flowing to the capture zone, resulting in an increase in the signal detected at the capture zone. Such assays provide reliable quantification of analyte when the analyte is present in low concentration.

The above-described assays suitable to quantify an analyte of interest present at low concentration are not suitable, however, to quantify an analyte of interestthat is present at high concentration. In such cases, the concentration of analyte may exceed the amount of labeled agent available to bind to the analyte, such that excess analyte is present. In these circumstances, excess analyte that is not bound by labeled agent competes with the label-antibody-analyte complex to bind to the capture agent in the capture zone. The capture agent in the capture zone will bind to un-labeled analyte (in other words, analyte not bound to a labeled agent) and to label-antibody-analyte complex. Un-labeled analyte that binds to the capture agent does not emit a detectable signal, however. As the concentration of analyte in the sample increases, the amount of un-labeled analyte that binds to the capture agent (in lieu of a label-antibody-analyte complex that emits a detectable signal) increases. As more and more un-labeled analyte binds to the capture agent in lieu of label-antibody-analyte complex, the signal detected at the capture zone decreases.

This phenomenon where the detected signal increases initially at low concentration and the detected signal decreases at high concentration is referred to as a "hook effect." As the concentration of analyte increases, more analyte binds to the labeled agent, resulting in increased signal strength. At saturated concentration, the labeled agent is saturated with analyte from the sample (for example, the available quantity of labeled agent has all or nearly all bound to analyte from the sample), and the detected signal has reached a maximum signal intensity. As the concentration of the analyte in the sample continues to increase beyond maximum signal intensity, there is a decrease in the detected signal as excess analyte above the labeled agent saturation point competes with the labeled agent-analyte to bind to the capture agent.

The hook effect, also referred to as "the prozone effect," adversely affects lateral flow assays, particularly in situations where the analyte of interest is present in the sample at elevated concentration. The hook effect can lead to inaccurate test results. For example, the hook effect can result in false negatives or inaccurately low results. Specifically, inaccurate results occur when a sample contains elevated levels of analyte that exceed the concentration of labeled agent deposited on the test strip. In this scenario, when the sample is placed on the test strip, the labeled agent becomes saturated, and not all of the analyte becomes labeled. The unlabeled analyte flows through the assay and binds at the capture zone, out-competing the labeled complex, and thereby reducing the detectable signal. Thus, the device (or the operator of the device) is unable to distinguish whether the optical signal corresponds to a low or a high concentration, as the single detected signal corresponds to both a low and a high concentration. If analyte levels are great enough, then the analyte completely out-competes the labeled complex, and no signal is observed at the capture zone, resulting in a false negative test result.

### Example Lateral Flow Devices that Accurately Quantify a Plurality of Analytes Present in a Single Sample at Both High and Low Concentrations

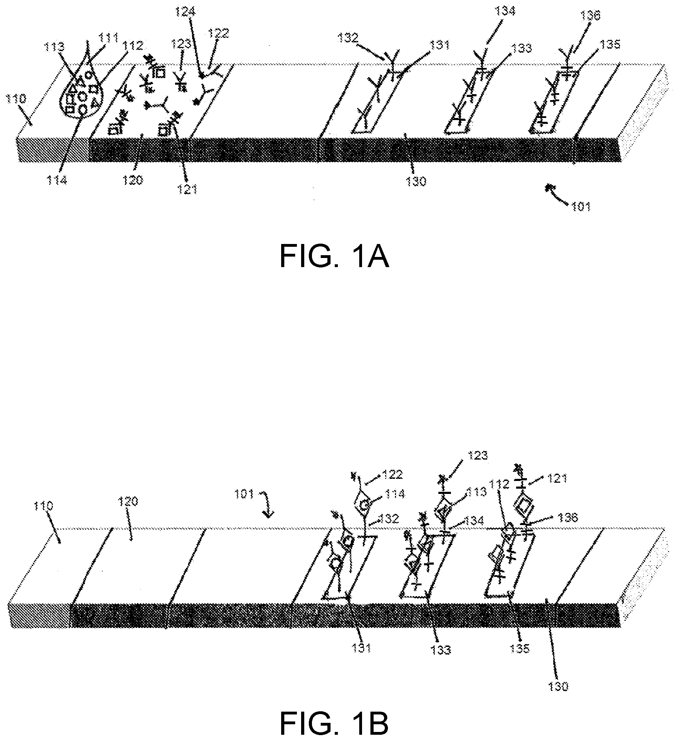

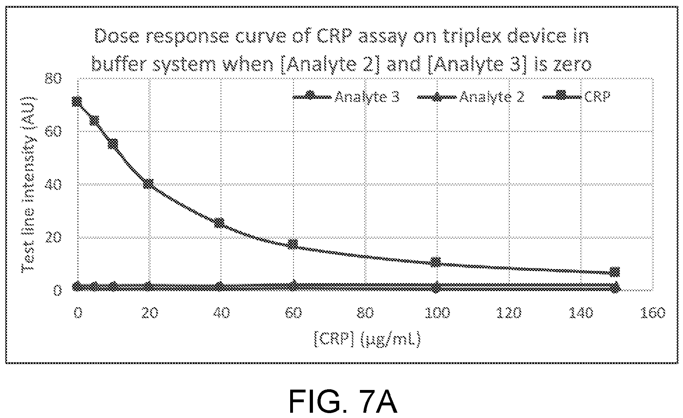

Lateral flow assays, test systems, and methods described herein address these and other drawbacks of multiplex sandwich-type lateral flow assays. Figures 1A-6B illustrate example lateral flow assays that can precisely measure a quantity of a plurality of analytes of interest, wherein one or more analytes of interest are present at high concentration and one or more analytes of interest are present at low concentration in a single sample. Figures 7A-7C provide example dose response curves that graphically illustrate the optical signal measured from the lateral flow assays described herein, and specifically the relationship between a magnitude of an optical signal detected at the capture zone (measured along the y-axis) and the concentration of analyte in the sample applied to the assay (measured along the x-axis). It will be understood that, although assays according to the present disclosure are described in the context of reflective-type labels generating optical signals, assays according to the present disclosure may include labels of any suitable material that are configured to generate fluorescence signals, magnetic signals, or any other detectable signal.

The lateral flow assay devices, systems, and methods described herein are capable of detecting the presence of and determining the concentration of a plurality of analytes in a sample, wherein one or more analytes are present in high concentration and one or more analytes are present in low concentration. In some embodiments, a first analyte of interest in the sample that is present in high concentration may be present in an amount of 10 million, 9 million, 8 million, 7 million, 6 million, 5 million, 4 million, 3 million, 2 million, 1 million, 500,000, 100,000, 50,000, 10,000, 5,000, 1,000, 500, 100, or 10 times greater than an amount of a second, different analyte of interest that is also present in the sample, but at low, very low, or extremely low concentration. In some cases, the second analyte of interest is present in minute quantities compared to the first analyte of interest in a given volume of fluid sample. For example, a high concentration analyte may be present in an amount of 10 to 100 µg/mL (10,000,000 to 100,000,000 pg/mL), whereas a low concentration analyte may be present in an amount of 10 to 100 pg/mL.

The example lateral flow assay 101 illustrated in Figures 1A-6B includes a test strip having a sample receiving zone 110, a label zone 120, and a detection zone 130, wherein the detection zone includes a first capture zone 135, a second capture zone 133, and a third capture zone 131. Figures 1A and 1B illustrate the lateral flow device 101 before and after a fluid sample 111 has been applied to a sample reservoir 110, wherein the fluid sample includes a first analyte of interest 112, a second analyte of interest 113, and a third analyte of interest 114. In the illustrated example, the label zone 120 is downstream of the sample receiving zone 110 along a direction of sample flow within the test strip. In some cases, the sample receiving zone 110 is located within and/or coextensive with the label zone 120. A first capture agent 136 is immobilized in the first capture zone 135, a second capture agent 134 is immobilized in the second capture zone 133, and a third capture agent 132 is immobilized in the third capture zone 131.

In implementations of the present disclosure, a first complex 121 is integrated on the label zone 120. The first complex 121 includes a label 124, a first antibody that specifically binds the first analyte of interest 112, and the analyte of interest 112. A second labeled antibody 123 is integrated on the label zone 120. The second labeled antibody 123 includes a label 124 and a second antibody that specifically binds the second analyte of interest 113. A third labeled antibody 122 is integrated on the label zone 120. The third labeled antibody 122 includes a label 124 and a third antibody that specifically binds the third analyte of interest 114. As illustrated in Figures 1A-6B, the label 124 is the same for each of the first complex 121, the second labeled antibody 123, and the third labeled antibody 122. It is to be understood that the label 124 may be identical for each of the first complex 121, the second labeled antibody 123, and the third labeled antibody 122. Alternatively, the label may be different for each of the first complex 121, the second labeled antibody 123, and the third labeled antibody 122. Thus, the label may provide the same or different optical signals for each of the plurality of analytes of interest. The label may be a reflective-type labels generating an optical signal, a latex bead label configured to generate fluorescence signals, a magnetic nanoparticle label configured to generate magnetic signals, or any other label configured to generate a detectable signal.

For example, a label may be any substance, compound, or particle that can be detected, such as by visual, fluorescent, radiation, or instrumental means. A label may be, for example, a pigment produced as a coloring agent or ink, such as Brilliant Blue, 3132 Fast Red 2R, and 4230 Malachite Blue Lake. A label may be a particulate label, such as, blue latex beads, gold nanoparticles, colored latex beads, magnetic particles, carbon nanoparticles, selenium nanoparticles, silver nanoparticles, quantum dots, up converting phosphors, organic fluorophores, textile dyes, enzymes, or liposomes.

In some cases, the first complex 121, the second labeled antibody 123, and the third labeled antibody 122 are formed and applied to the test strip prior to use of the test strip by an operator. For example, the first complex 121, the second labeled antibody 123, and the third labeled antibody 122 can be integrated in the label zone 120 during manufacture of the test strip. In another example, the first complex 121, the second labeled antibody 123, and the third labeled antibody 122 are integrated in the label zone 120 after manufacture but prior to application of the fluid sample 111 to the test strip. The first complex 121, the second labeled antibody 123, and the third labeled antibody 122 can be integrated into the test strip in a number of ways discussed in greater detail below.

Accordingly, in embodiments of the lateral flow device of the present disclosure, the first complex 121, the second labeled antibody 123, and the third labeled antibody 122 are formed and integrated on the test strip before any fluid sample 111 has been applied to the lateral flow device 101. In one non-limiting example, the first complex 121, the second labeled antibody 123, and the third labeled antibody 122 are formed and integrated onto the conjugate pad of the test strip before any fluid sample 111 is applied to the lateral flow device 101. Further, in embodiments of the lateral flow device of the present disclosure, the analyte in first complex 121 is not analyte from the fluid sample 111.

To perform a test using the test strip 101, a sample 111 having a first analyte of interest 112, a second analyte of interest 113, and a third analyte of interest 114, as shown in Figures 1A and 1B, is deposited on the sample receiving zone 110. In the illustrated embodiment where the label zone 120 is downstream of the sample receiving zone 110, first analyte of interest 112, second analyte of interest 113, and third analyte of interest 114 in the sample 111 flows to the label zone 120 and comes into contact with the integrated first complex 121, the second labeled antibody 123, and the third labeled antibody 122. The sample 111 solubilizes the first complex 121, the second labeled antibody 123, and the third labeled antibody 122. In one non-limiting example, the sample 111 dissolves the first complex 121, the second labeled antibody 123, and the third labeled antibody 122. The bonds that held the first complex 121, the second labeled antibody 123, and the third labeled antibody 122 to the surface of the test strip in the label zone 120 are released, so that the first complex 121, the second labeled antibody 123, and the third labeled antibody 122 are no longer integrated onto the surface of the test strip. The second labeled antibody 123 binds to the second analyte of interest 113 in the sample forming a second complex, and the third labeled antibody 122 binds to the third analyte of interest 114 in the sample forming a third complex.

The first complex 121, the second complex, and the third complex migrate with first analyte 112 (which is unbound) in the sample 111 along the fluid front to the detection zone 130. First capture agent 136 at the first capture zone 135 binds to first complex 121 and first analyte 112 from the sample 111. The second capture agent 134 at the second capture zone 133 binds to the second complex, and the third capture agent 132 at the third capture zone 131 binds to the third complex.

In implementations of the present disclosure, depending on the quantity of first analyte 112 in the sample 111, the first complex 121 and the first analyte 112 compete with each other to bind to first capture agent 136 in the first capture zone 135. A first detectable signal is detected at the first capture zone 135, wherein the first detectable signal decreases from a signal of maximum intensity in the presence of a first analyte of interest 112 in the sample, because the first analyte of interest 112 competes with the first complex 121 for binding to the first capture agent 136 at the first capture zone. Conversely, a second detectable signal is detected at the second capture zone 133, and increases in intensity with increasing concentrations of the second analyte of interest 113 in the sample, because the second analyte of interest 113 forms a second complex that emits a detectable signal at the second capture zone 133. Similarly, a third detectable signal is detected at the third capture zone 131, and increases in intensity with increasing concentrations of the third analyte of interest 114 in the sample, because the third analyte of interest 114 forms a third complex that emits a detectable signal at the third capture zone 131.

Accordingly, lateral flow devices according to the present disclosure include a first complex including a label, a first antibody that specifically binds the first analyte of interest, and the first analyte of interest; a second labeled antibody that specifically binds a second analyte of interest; and a third labeled antibody that specifically binds a third analyte of interest, each of which are bound to a label zone of the lateral flow device in a first phase (for example, prior to application of the fluid sample to the lateral flow device), and then migrate through the test strip in a second, later phase (for example, upon application of the fluid sample to the sample receiving zone). The first complex can bind to a first capture agent in the first capture zone, the second complex can bind to a second capture agent in the second capture zone, and the third complex can bind to a third capture agent in the third capture zone in a third phase (for example, after the fluid sample has flowed to the detection zone). Thus, the first complex, the second labeled antibody, and the third labeled antibody described herein can be initially positioned in a first region (such as a label zone) of a lateral flow device, then (upon contact with a fluid), migrate with the fluid to other regions of the lateral flow device downstream of the first region, and then bind to capture agents in the capture zone.

As described above, the fluid sample 111 solubilizes the first complex 121, the second labeled antibody 123, and the third labeled antibody 122. In one implementation, the first analyte of interest 112 in the sample 111 does not interact with, or does not interact substantially with, the first complex 121 during this process. Without being bound to any particular theory, in this implementation of the lateral flow devices described herein, the first analyte of interest 112 does not conjugate to, bind to, or associate with the first complex 121 as the sample 111 flows through the label zone 120. In another implementation of the lateral flow devices described herein, the first analyte of interest 112 in the sample 111 interacts with the first complex 121 when the fluid sample 111 solubilizes the first complex 121. In one non-limiting example, and without being bound to any particular theory, at least some first analyte of interest 112 in the sample 111 exchanges with first analyte present in the first complex 121. Without being bound to any particular theory, in this implementation, first capture agent 136 in the first capture zone 135 may bind to at least some first complex 121 where the analyte in the first complex 121 is first analyte of interest 112 introduced onto the device 101 via the sample 111.

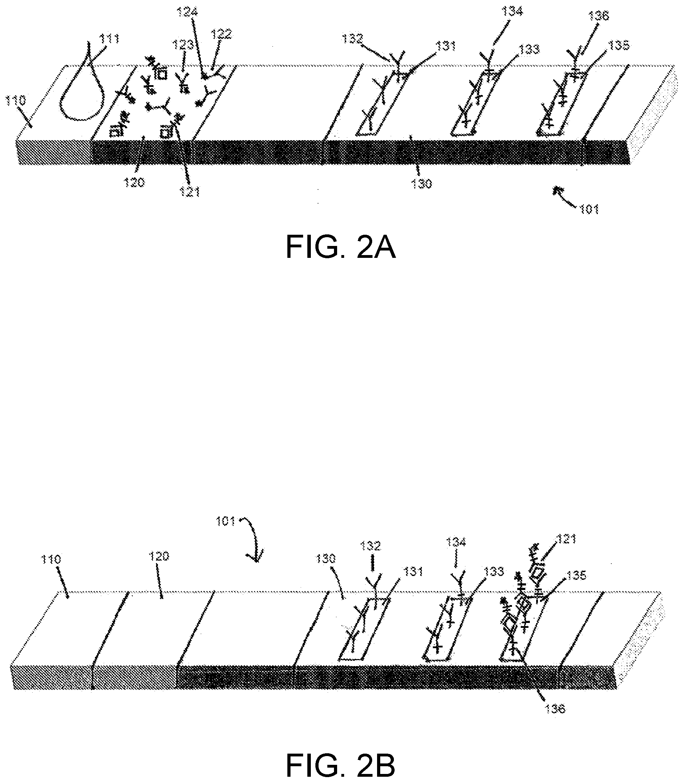

When a first analyte of interest 112, a second analyte of interest 113, and a third analyte of interest 114, are each absent from the fluid sample 111 (or they are present below a detectable level) as shown in Figures 2A and 2B, the first complex 121 saturates the first capture agent 136 at the first capture zone 135 (for example, every first capture agent 136 molecule in the first capture zone 135 binds to one first complex 121 that flowed from the label zone 120). The second capture agent 134 in the second capture zone 133 does not bind to any second complex because second complex does not form in the absence of the second analyte of interest 113. In situations where the second analyte of interest 113 is present below the detectable level, no detectable amount of second complex forms. The third capture agent 132 in the third capture zone 131 does not bind to any third complex because third complex does not form in the absence of the third analyte of interest 114. In situations where the third analyte of interest 114 is present below the detectable level, no detectable amount of third complex forms. The first complex 121 captured in the first capture zone 135 emits a first detectable optical signal that is the maximum intensity signal that can be obtained from the first capture zone 135 of the lateral flow device 101. The first optical signal detected at the first capture zone 135 in a scenario where no first analyte of interest 112 is present (or less than the detectable level is present) in the sample 111 is a maximum intensity signal at the first capture zone, because every available first capture agent 136 at the first capture zone 135 has bound to a first complex 121. In the absence of (or less than the detectable level of) a second analyte of interest 113, no second complex is formed (or no detectable amount of second complex is formed), and thus the second capture agent 134 does not capture any second complex (or any detectable amount of second complex), and no second detectable signal is observed. Similarly, in the absence of (or less than the detectable level of) a third analyte of interest 114, no third complex is formed (or no detectable amount of third complex is formed), and thus the third capture agent 132 does not capture any third complex (or any detectable amount of third complex), and no third detectable signal is observed.

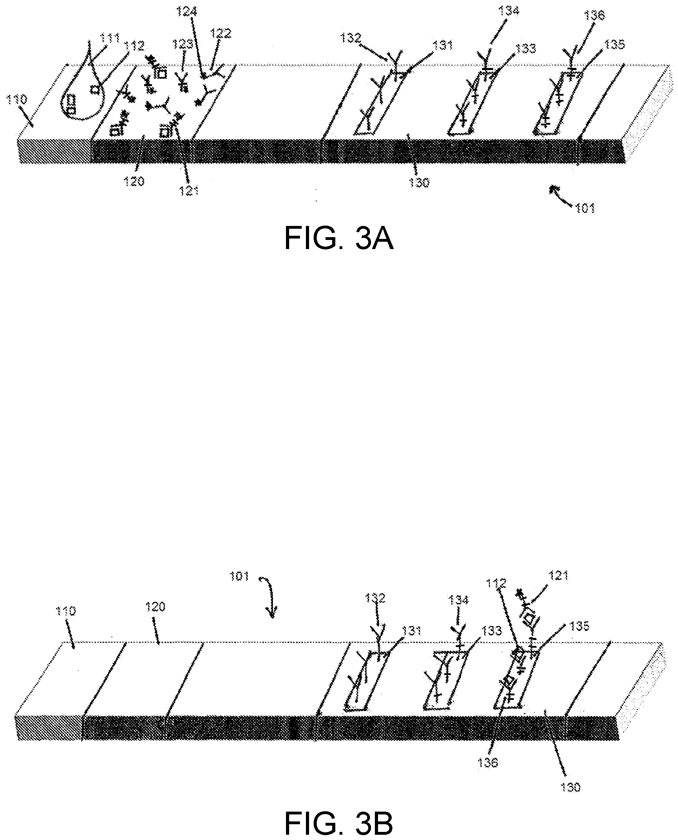

Figures 3A-3B illustrate an example lateral flow assay where only a first analyte of interest 112 is present in the fluid sample 111, but the second analyte of interest 113 and the third analyte of interest 114 are not present or are present below the detectable level in the fluid sample 111. In this example, the first analyte of interest 112 competes with first complex 121 for binding to the first capture agent 136 at the first capture zone 135. The result is increased quantities of the first analyte of interest 112 being bound by first capture agent 136 at the first capture zone 135 as the concentration of first analyte of interest 112 increases in the sample 111. Because the first analyte of interest 112 does not emit a detectable signal, and because fewer first complex 121 binds to first capture agent 136 at the first capture zone 135 in the presence of first analyte of interest 112, a first detectable signal is decreased in comparison to a maximum signal intensity that is observed when first analyte of interest 112 is absent from the sample 111.

An exemplary dose response curve depicting the example lateral flow assay of Figures 3A and 3B is shown in Figure 7A. In Figure 7A, the signal intensity for a first analyte of interest (here, signal intensity measured from the first capture zone configured to bind with CRP plotted with squares) detected at the first capture zone decreases with increasing concentrations of the first analyte of interest in the sample. In contrast, the second signal for the second analyte of interest (here, signal intensity measured from the second capture zone configured to bind with Analyte 2 plotted with triangles) and the third signal for the third analyte of interest (here, signal intensity measured from the third capture zone configured to bind with Analyte 3 plotted with circles) do not increase because of the absence of (or less than the detectable level of) the second analyte of interest and the third analyte of interest in the sample.

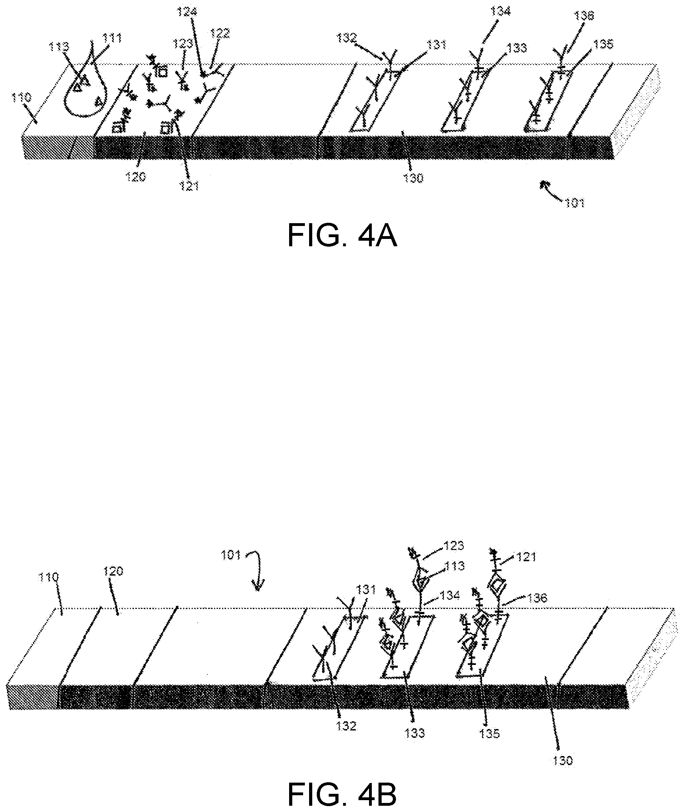

Figures 4A-4B illustrate an example lateral flow assay where only a second analyte of interest 113 is present in the fluid sample 111, but the first analyte of interest 112 and the third analyte of interest 114 are not present or are present below the detectable level in the fluid sample 111. In this example, second analyte of interest 113 binds to second labeled antibody 123 that specifically binds to the second analyte of interest 113, forming a second complex. The second complex flows with the fluid sample 111 to the detection zone 130, where the second complex is bound by second capture agent 134 at the second capture zone 133. A second detectable signal is emitted from the second complex bound at the second capture zone 133, indicating the presence of second analyte of interest 113 in the fluid sample 111. As the concentration of the second analyte of interest 113 increases in the sample 111, the intensity of the second detectable signal emitted from the second complex bound at the second capture zone 133 increases.

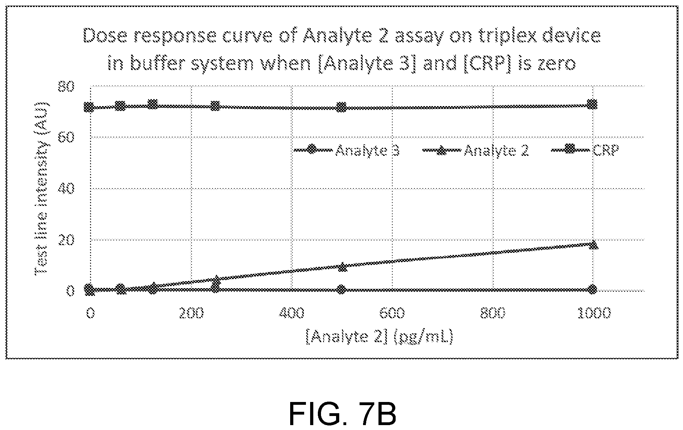

An exemplary dose response curve depicting the example lateral flow assay of Figures 4A and 4B is depicted in Figure 7B. In Figure 7B, signal intensity for a second analyte of interest (here, signal intensity measured from the second capture zone configured to bind with Analyte 2 plotted with triangles) increases with an increase in concentration of the second analyte of interest in the sample. The signal intensity for the first analyte of interest (here, signal intensity measured from the first capture zone configured to bind with CRP plotted with squares) remains at or substantially at a maximum value (in this example, around 70 AU (arbitrary signal intensity units)) for all concentrations of the second analyte of interest, indicating an absence of (or less than the detectable level of) the first analyte of interest in the sample. The signal intensity for the third analyte of interest (here, signal intensity measured from the third capture zone configured to bind with Analyte 3 plotted with circles) does not increase, indicating an absence of (or less than the detectable level of) the third analyte of interest in the sample.

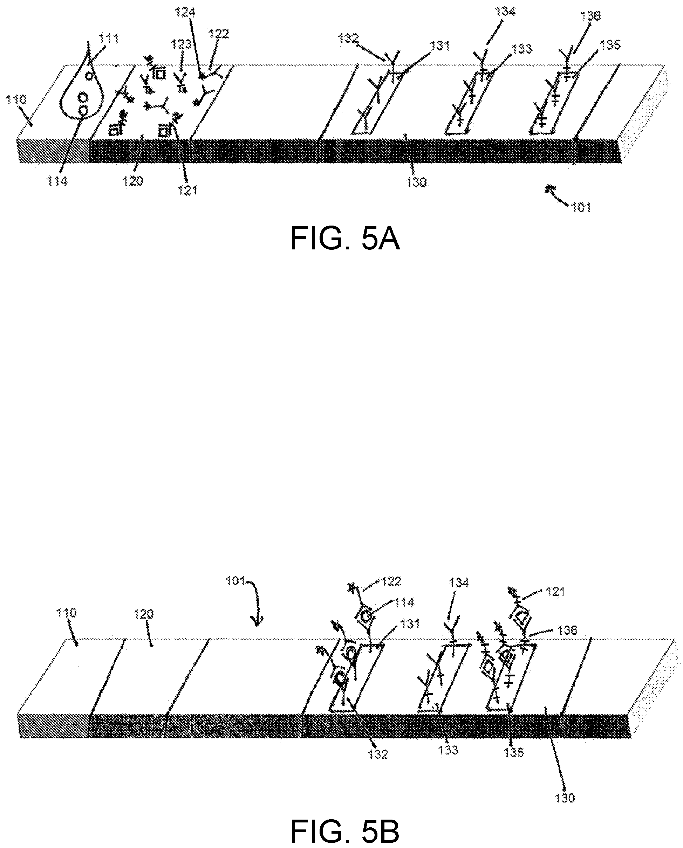

Figures 5A-5B illustrate an example lateral flow assay where only a third analyte of interest 114 is present in the fluid sample 111, but the second analyte of interest 113 and the first analyte of interest 112 are not present or are present below the detectable level in the fluid sample 111. In this example, third analyte of interest 114 binds to the third labeled antibody 122 that specifically binds to the third analyte of interest 114, forming a third complex. The third complex flows with the fluid sample 111 to the detection zone 130, where the third complex is bound by the third capture agent 132 at the third capture zone 131. A third detectable signal is emitted from the third complex bound at the third capture zone 131, indicating the presence of third analyte of interest 114 in the fluid sample 111. As the concentration of the third analyte of interest 114 increases in the sample 111, the intensity of the third detectable signal emitted from the third complex bound at the third capture zone 131 increases.

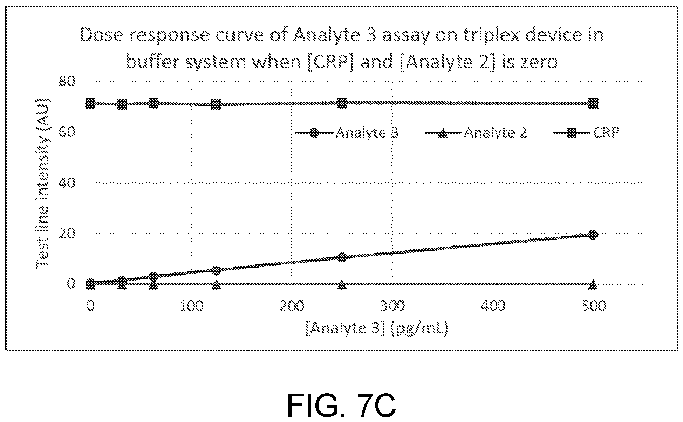

An exemplary dose response curve depicting the example lateral flow assay of Figures 5A and 5B is depicted in Figure 7C. In Figure 7C, signal intensity for a third analyte of interest (here, signal intensity measured from the third capture zone configured to bind with Analyte 3 plotted with circles) increases with an increase in concentration of the third analyte of interest in the sample. The signal intensity for the first analyte of interest (here, signal intensity measured from the first capture zone configured to bind with CRP plotted with squares) remains at or substantially at a maximum value (in this example, around 70 AU for all concentrations of the third analyte of interest, indicating an absence of (or less than the detectable level of) the first analyte of interest in the sample. The signal intensity for the second analyte of interest (here, signal intensity measured from the second capture zone configured to bind with Analyte 2 plotted with triangles) does not increase, indicating an absence of (or less than the detectable level of) the second analyte of interest in the sample.

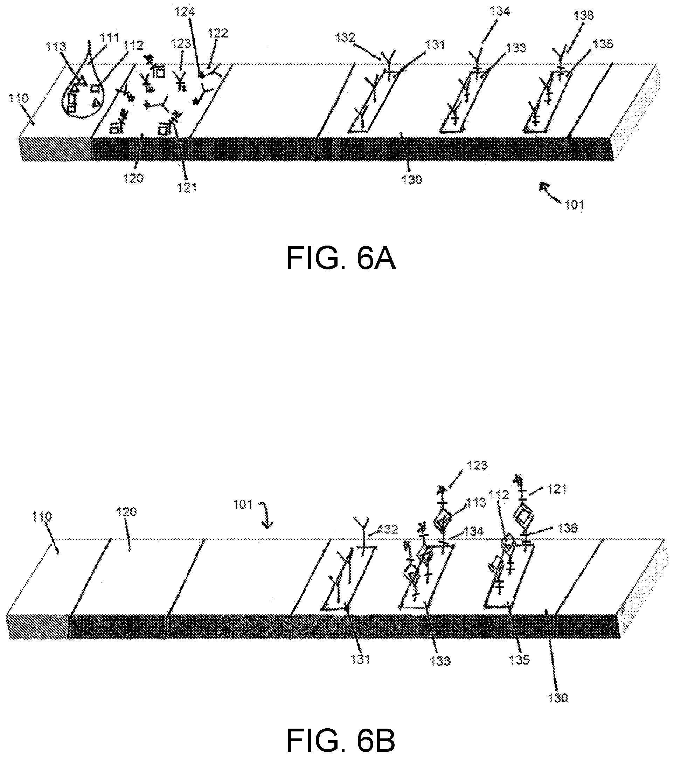

Figures 6A-6B illustrate an example lateral flow assay where only the first analyte of interest 112 and the second analyte of interest 113 are present in the fluid sample 111, but the third analyte of interest 114 is not present or is present below the detectable level in the fluid sample 111. This example lateral flow assay is a combination of Figures 3A and 3B with Figures 4A and 4B, illustrating an iteration where more than one analyte of interest may be present, but where all analytes of interest are not necessarily present (or not necessarily present at the detectable level). In this example, the first analyte of interest 112 in the sample competes with the first complex 121 for binding to the first capture agent 136 at first capture zone 135 in a manner described above with reference to Figures 3A and 3B. A first detectable signal detected at the first capture zone 135 decreases with increasing concentration of the first analyte of interest 112 from a maximum signal intensity, indicating the presence and quantity of first analyte of interest 112 in the fluid sample 111. Simultaneously or near simultaneously, the second analyte of interest 113 binds with the second labeled antibody 123 in the label zone, forming a second complex. The second complex flows to the detection zone and binds to the second capture agent 134 at the second capture zone 133. A second detectable signal increases with increasing concentration of second analyte of interest 113, indicating the presence and quantity of the second analyte of interest 113 in the fluid sample 111.

Figures 1A-6B illustrate the first capture zone 135, the second capture zone 133, and the third capture zone 131 arranged perpendicular to a longitudinal axis of the test strip, with the first capture zone 135 furthest from the sample receiving zone 110 and the third capture zone 131 closest to the sample receiving zone 110. In this non-limiting example, the first complex 121 would flow through the third capture zone 131 and the second capture zone 133 before reaching the first capture zone 135 and binding to the first capture agent 136 immobilized on the first capture zone 135. These figures are illustrative, and various iterations, alterations, and modifications may be realized. The relative positions of the first capture zone 135, the second capture zone 133, and the third capture zone 131 may differ from that illustrated in Figures 1A-6B such that the fluid sample 111 flows through the capture zones in a different sequence than that illustrated. For example, the first capture zone, the second capture zone, and the third capture zone may be arranged perpendicular to a longitudinal axis of the test strip in various sequenced orders (for example 3, 2, 1; 3, 1, 2; 1, 2, 3; 1, 3, 2; 2, 1, 3; or 2, 3, 1). Furthermore, the capture zones may be placed parallel to rather than perpendicular to a longitudinal axis of the test strip, such that each capture zone is equally distant from the sample receiving zone.

There are many methods to determine the maximum intensity signal of the first capture zone 135 of the lateral flow device 101. In one non-limiting example, the maximum intensity signal that can be obtained from a particular first capture zone 135 of the lateral flow device 101 can be determined empirically and stored in a look-up table. In some cases, the maximum intensity signal is determined empirically by testing lateral flow devices 101 of known features and construction, for example by averaging the maximum intensity signal obtained when a sample having a zero or almost zero concentration of the first analyte of interest is applied to lateral flow devices 101 of known specifications and construction. In another non-limiting example, the maximum intensity signal that can be obtained from a particular first capture zone 135 of the lateral flow device 101 can be determined using theoretical calculations given the known specifications and construction of the lateral flow device 101 (such as, for example, the amount and specific characteristics of the first complex 121 integrated on the label zone 120).

Further, it will be understood that although reference is made herein to "maximum intensity signal," signals that are within a particular range of the expected maximum intensity can be deemed substantially equivalent to the "maximum intensity signal." In addition, it will be understood that "maximum intensity signal" may refer to a maximum intensity optical signal, maximum intensity fluorescence signal, maximum intensity magnetic signal, or any other type of signal occurring at maximum intensity. As one non-limiting example, a detected signal at the first capture zone 135 that is within 1% of the expected maximum intensity signal is deemed substantially equivalent to the expected maximum intensity signal at the first capture zone 135. If the maximum intensity signal is at or about 70 AU, a detected signal within a range of about 75.3 AU to about 70.7 AU would be deemed substantially equivalent to the maximum intensity signal of 70 AU. As another example, in the non-limiting embodiment described with reference to Figures 7A-7C, a detected signal at the first capture zone 135 that is within 10% of the expected maximum intensity signal is deemed substantially equivalent to the expected maximum intensity signal at the first capture zone 135. Thus, in the example illustrated in Figures 7A-7C where the maximum intensity signal is at or about 70 AU, a detected signal within the range of about 63 AU to about 77 AU is deemed substantially equivalent to the maximum intensity signal of 70 AU. These examples are provided for illustrative purposes only, as other variances may be acceptable. For instance, in lateral flow assay device according to the present disclosure, a detected signal at the first capture zone 135 that is within any suitable range of variance from the expected maximum intensity signal (such as but not limited to within 1.1%, 1.2%, 1.3%, 1.4%, 1.5%, 2.0%, 2.5%, 3%, 3.5%, 4%, 4.5%, 5%, 5.5%, 6%, 6.5%, 7%, 7.5%, 8%, 8.5%, 9%, 9.5%, 10%, 11%, 12%, 13%, 14%, 15% of the expected maximum intensity signal) can be deemed substantially equivalent to the expected maximum intensity signal at the first capture zone 135.

As illustrated in Figure 7A, the decrease in the first signal at the first capture zone 135 as the concentration of first analyte of interest increases is advantageously gradual in embodiments of lateral flow devices according to the present disclosure. As a result of this gradual decrease in the detected first signal, embodiments of lateral flow devices described herein advantageously allow a detector to precisely measure the first signal with high resolution and a data analyzer to determine, with high precision, the concentration of the first analyte of interest when the concentration is high.

In addition, the dose response curve with respect to an analyte of interest present at high concentration in lateral flow devices according to the present disclosure advantageously begins at a maximum intensity signal and then decreases from this maximum intensity signal. This means that, advantageously, in the dose response curve for a first analyte present at high concentration, no signal in the portion of the dose response curve where the signal is decreasing will have a magnitude that is the same as the maximum intensity signal. Further, because the first signal when the concentration of the first analyte in the sample is low will be the same as or effectively the same as the maximum intensity signal (for example, they are deemed substantially equivalent to the maximum intensity signals as described above), there is a plateau of first optical signals at a relatively constant value ("maximum intensity signal") for zero to low concentrations of first analyte (as will be discussed in detail below with reference to non-limiting examples). This means that, advantageously, no signal in the portion of the first dose response curve where the first signal is decreasing will have a magnitude that is about the same as the maximum intensity signal. False negatives and inaccurately low readings are thus avoided with respect to analytes present in high concentration in embodiments of the lateral flow devices described herein, and allows for detection of both high concentration analytes and low concentration analytes present in a single sample without diluting or other pre-processing the sample prior to application to a single lateral flow assay.

Advantageously, in embodiments of lateral flow devices described herein, the first complex 121 can be pre-formulated to include a known quantity of first analyte of interest prior to deposition on the conjugate pad. In some embodiments, first analyte of interest of a known concentration is incubated with an antibody or fragment of an antibody and label molecules in a reaction vessel that is separate from the test strip. During incubation, the first analyte of interest becomes conjugated to, bound to, or associated with the antibody and label molecules to form a first complex 121 as described above. After incubation, the first complex 121 is either directly added to a solution at a precise, known concentration or isolated to remove excess free first analyte of interest before being sprayed onto the conjugate pad. The solution including the first complex 121 is applied to the test strip, such as on the label zone 120 described above. During deposition, the first complex 121 becomes integrated on the surface of the test strip. In one non-limiting example, the first complex 121 is integrated onto the conjugate pad of the test strip. Advantageously, first complex 121 can remain physically bound to and chemically stable on the surface of the test strip until an operator applies a fluid sample to the test strip, whereupon the first complex 121 unbinds from the test strip and flows with the fluid sample as described above.

Similarly, second labeled antibody 123 and third labeled antibody 122 may be separately formulated. For example, a second antibody that specifically binds a second analyte of interest may be incubated with label molecules, thereby forming second labeled antibody 123. The second labeled antibody 123 can be deposited on the test strip similar to deposition of the first complex 121, or in any other suitable manner. The second labeled antibody 123 can remain physically bound to and chemically stable on the surface of the test strip until an operator applies a fluid sample to the test strip, whereupon the second labeled antibody 123 unbinds from the test strip, binds to any second analyte present in the fluid sample, and flows with the fluid sample as described above. Similar methods may be used for a third labeled antibody, or any additional labeled antibody or complex for detection of additional analytes of interest.

In some embodiments, the first complex 121, the second labeled antibody 123, and the third labeled antibody 122 are each deposited in an amount ranging from about 0.1-20 µL/test strip. In some embodiments, the first complex 121, the second labeled antibody 123, and the third labeled antibody 122 are each deposited in an amount of 0.1, 0.2, 0.3, 0.4, 0.5, 0.6, 0.7, 0.8, 0.9, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, 10, 11, 12, 13, 14, 15, 16, 17, 18, 19, or 20 µL/test strip in the label zone. In one non-limiting example, the first complex 121 was deposited in an amount of about 3 µL/cm, the second labeled antibody 123 was deposited in an amount of about 7 µL/cm, and the third labeled antibody 122 was deposited in an amount of about 7 µL/cm.

A solution including the first complex 121, a solution including the second labeled antibody 123, and a solution including the third labeled antibody 122, can be applied to the test strip in many different ways. In one example, the solutions are applied to the label zone 120 by spraying the solutions with airjet techniques. In another example, the solutions are deposited by pouring the solutions, spraying the solutions, formulating the solutions as a powder or gel that is placed or rubbed on the test strip, or any other suitable method to apply the first complex 121, the second labeled antibody 123, and the third labeled antibody 122. In some embodiments, after deposition, the first complex 121, the second labeled antibody 123, and the third labeled antibody 122 are dried on the surface of the test strip after deposition by heating or blowing air on the conjugate pad. Other mechanisms to dry the first complex 121, the second labeled antibody 123, and the third labeled antibody 122 on the surface of the test strip are suitable. For example, vacuum or lyophilization can also be used to dry the first complex 121, the second labeled antibody 123, and the third labeled antibody 122 on the conjugate pad.

In some cases, the first complex 121, the second labeled antibody 123, and the third labeled antibody 122 are not added to a solution prior to deposition and are instead applied directly to the test strip. The first complex 121, the second labeled antibody 123, and the third labeled antibody 122 can be directly applied using any suitable method, including but not limited to applying compressive or vacuum pressure to the first complex 121, the second labeled antibody 123, and the third labeled antibody 122 on the surface of the test strip and/or applying first complex 121, second labeled antibody 123, and third labeled antibody 122 in the form of lyophilized particles to the surface of the test strip.

Embodiments of the lateral flow assay described herein need not include a control line or zone configured to confirm that a sample applied in the sample receiving zone 110 has flowed to the detection zone 130 as intended. Under normal operating circumstances, some detectable signal will always be emitted from the first capture zone 135 if the sample has flowed to the first capture zone 135. Advantageously, the first capture zone 135 can be positioned downstream of both the third capture zone 131 and the second capture zone 133 and visually indicate that the sample 111 has flowed through all three capture zones as intended, such that the first capture zone 135 effectively functions as a control line or zone. Under normal operating circumstances detectable signal will always be emitted from the first capture zone 135 if the sample has flowed to the first capture zone 135, even if the first analyte of interest is present in the sample at extremely low concentrations. This is because the lateral flow devices of the present disclosure generate a first dose response curve that remains at or near a maximum intensity signal for zero or low concentrations of the first analyte of interest. Even in the presence of physiologically possible high concentration of the first analyte 112 in the sample, the signal on the first capture zone 135 may significantly decrease but not completely disappear by careful design of the lateral flow assay. Therefore, the absence of any detectable signal at the first capture zone 135 after the sample has been applied to the sample receiving zone 110 can be used an indication that the lateral flow assay did not operate as intended (for example, the sample did not flow to the first capture zone 135 as intended, or as another example, the immobilized first capture agents 136 at the first capture zone 135 are defective or faulty). Accordingly, a further advantage of embodiments of lateral flow devices according to the present disclosure is the ability of the first capture zone 135 to function as a control line, thereby permitting a separate control line to be omitted from the test strip altogether. It will be understood, however, that a control line could be included in embodiments of lateral flow devices described herein for a variety of purposes, including but not limited to a viewing line, for normalizing noise, or for detecting interference from analytes in serum.

In some cases, the lateral flow device includes one or more control zones. The control zones may be in the detection zone or separate from the detection zone. In some embodiments, a control zone may be a positive control zone, which may include small molecules conjugated with a protein, such as bovine serum albumin (BSA). Positive control labeled antibody that specifically binds small molecules may be deposited on the conjugate pad. When positive control labeled antibody is rehydrated with a liquid sample it flows towards the positive control zone and binds to the small molecules forming a semi-sandwich. A positive control signal generated at the positive control zone is independent of the presence and concentration of the plurality of analytes present in the fluid sample, and therefore maintains relatively constant intensity. However, due to the variation of the amount of positive control labeled antibody deposited on the conjugate pad caused by uneven pad material, the intensity of the positive control signal generated at the positive control zone and the intensity of the signals generated at each capture zone may vary slightly from device to device even tested with the same sample. The change in intensity from device to device of signal at the positive control zone and capture zones are the same. Therefore, the positive control zone can be used as a reference line to better measure the relative signal intensities generated at the capture zones and hence the positive control zone may provide more accurate analyte concentration.

A lateral flow assay may additionally include a negative control zone. The negative control zone may include a negative control antibody from the same species as the antibodies used in the capture zones. Some components from some blood samples may interfere with immunoassay. If such an interfering substance does exist in one sample, it will not only interfere with the signal intensity at the capture zones, but also interfere with the signal intensity at the negative control zone. Embodiments of readers and data analyzers disclosed herein can process the signal measurements obtained from the negative control zone to either correct any calculation or notify an operator of an invalid result.

The following non-limiting examples illustrate features of lateral flow devices, test systems, and methods described herein, and are in no way intended to limit the scope of the present disclosure.

### Example 1

### Preparation of a Lateral Flow Assay to Quantify Proteins at both High and Low Concentration

The following example describes preparation of a lateral flow assay to quantify a plurality of analytes of interest as described herein. In this non-limiting example, the analytes of interest are proteins in a single sample: C-reactive protein (CRP), Analyte 2 (such as but not limited to procalcitonin (PCT)), and Analyte 3 (such as but not limited to interferon-induced GTP-binding protein Mx1). In this non-limiting example, CRP is present in a serum sample at an elevated or high concentration, whereas Analyte 2 or Analyte 3 are present in the serum sample at a low concentration.

CRP is a protein found in blood plasma. Levels of CRP rise in response to inflammation and infection. CRP is thus a marker for inflammation and infection that can be used to diagnose inflammation and infection. Elevated levels of CRP in the serum of a subject can be correlated to inflammation and/or bacterial infection in the subject. Normal levels of CRP in healthy human subjects range from about 1 µg/mL to about 10 µg/mL. Concentrations of CRP during mild inflammation and bacterial infection range from 10-40 µg/mL; during active inflammation and bacterial infection from 40-200 µg/mL; and in severe bacterial infections and burn cases greater than 200 µg/mL.Measuring and charting CRP levels be useful in determining disease progress or the effectiveness of treatments.

CRP is thus present in blood plasma across a large dynamic range, for example from low concentrations of about 1 µg/mL to about 10 µg/mL to very high concentrations of greater than 200 µg/mL. Although CRP can in some cases be measured with a high degree of sensitivity, such measurements typically have low specificity (for example, measuring CRP may be very sensitive to minute changes in concentration, but a single concentration measurement may correlate to more than one disease state or even no disease state (inflammation or other non-disease condition)). Embodiments of lateral flow devices, test systems, and methods described herein advantageously allow CRP to be measured with very high sensitivity while simultaneously measuring the concentration of analytes of interest that are present at low concentration in the same single sample, to thereby increase the specificity of the multiplex assay as a whole. Embodiments of the present disclosure thus measure, very accurately, the concentration of CRP across its large dynamic range alongside the concentration of additional analytes of interest present at one-millionth the concentration of CRP, using a single sample applied to a single lateral flow assay in a single test event, including in situations where the single sample is not diluted or pre-processed prior to application to the single assay. For example, the single sample may be an undiluted, whole blood sample; an undiluted venous blood sample; an undiluted capillary blood sample; an undiluted, serum sample; and an undiluted plasma sample.

Analyte 2 is a protein that is elevated in the blood during a viral infection. One non-limiting example of Analyte 2 is PCT. Serum concentration of PCT is normally around < 0.05 ng/mL, but in circumstances of systemic inflammation, particularly bacterial infection, PCT is produced in large quantities by many body tissues. In some cases of sepsis, PCT may be present at a concentration of 0.5 - 2 ng/mL. The following examples are not limited to PCT, however, and will refer to a second analyte of interest as Analyte 2.

Analyte 3 is a protein that is elevated in the blood plasma during a viral infection. One non-limiting example of Analyte 3 is Mx1. The following examples are not limited to Mx1, however, and will refer to a third analyte of interest as Analyte 3.

An assay for determining the concentration or presence of each of CRP, Analyte 2, Analyte 3 requires detection of analytes at low concentrations and simultaneous detection of analytes at high concentrations. Indeed, CRP concentrations may be one million times greater than the concentration of Analyte 2 and Analyte 3. Furthermore, detection of mild increase of CRP, increase of Analyte 3, and Analyte 2 can be indicative of a viral infection. Detection of increased level of CRP and Analyte 2, but not Analyte 3, can be indicative of a bacterial infection. Detection of increased level of CRP only, with the absence of Analyte 2 and Analyte 3 detection can be indicative of inflammation. Detection of none of CRP, Analyte 2, or Analyte 3 (or detection of CRP at within the range of a healthy subject of about 1 µg/mL to about 10 µg/mL) can be a negative result for infection, indicating that it is unlikely that the subject suffers from a viral or bacterial infection.

The assay prepared according to this non-limiting example can be used to determine the presence and concentration of CRP, Analyte 2, and Analyte 3 (the analytes of interest) in a whole blood or fraction of whole blood sample even when the concentration of CRP is high and the concentrations of Analyte 2 and Analyte 3 are low. The assay includes a complex that includes a label, a first antibody or fragment thereof that specifically binds CRP, and CRP. The assay further includes a labeled second antibody or fragment thereof that specifically binds Analyte 2 and a labeled third antibody or fragment thereof that specifically binds Analyte 3.

To prepare the assay, anti-CRP antibody was incubated with gold nanoparticles to form labeled anti-CRP antibody. The labeled antibody was incubated with CRP to form a complex of labeled antibody bound to CRP. The complex was deposited in an amount of 1.8 µL/test strip onto a conjugate pad (label zone) by spraying a solution including the complex with airjet.

Anti-Analyte2 antibody was incubated with gold nanoparticles to form labeled anti-Analyte2 antibody. The labeled anti-Analyte2 antibody was deposited in an amount of 7 µL/test strip onto a conjugate pad (label zone) by spraying a solution including the labeled anti-Analyte2 antibody with airjet. Anti-Analyte3 antibody was incubated with gold nanoparticles to form labeled anti-Analyte3 antibody. The labeled anti-Analyte3 antibody was deposited in an amount of 7 µL/test strip onto a conjugate pad (label zone) by spraying a solution including the labeled anti-Analyte3 antibody with airjet. The conjugate pad was heated to dry the complex and each of labeled anti-Analyte2 antibody and labeled anti-Analyte3 antibody to the conjugate pad.

The amount of antibody-label-CRP complex deposited on the conjugate pad was carefully considered to ensure a requisite amount of complex to provide an optimal range of optical signals at the capture zone that will allow a test system to quantify elevated levels of CRP. Depositing an excess amount of complex on the conjugate pad will shift the dose response curve, such that the quantifiable concentration of CRP is excessively high (potentially generating optical signals for very high concentrations of CRP (if present) but not generating optical signals for mild to high concentrations). Depositing an insufficient amount of complex on the conjugate pad shifts the dose response curve in the other direction, resulting in signals that may not allow quantification of very high CRP concentrations but quantification of relatively low CRP concentration.

In this example, the optimal amount of antibody-label-CRP complex to add to the conjugate pad results in 50 ng of CRP deposited on the conjugate pad, corresponding to a signal of 70.06 AU. At this amount, the ratio of unlabeled CRP in the sample to antibody-label-CRP complex as they compete to bind to the capture agent in the capture zone generates a strong optical signal over an optimal range of unlabeled CRP concentrations, thereby allowing for adequate resolution of the signal, and elevated CRP concentration in a sample can be accurately quantified. In addition, the amount of labeled anti-Analyte2 antibody and labeled anti-Mx1 antibody deposited on the conjugate pad was about 260 ng per test strip.

In addition, the assay was prepared having a detection zone. The detection zone includes a capture zone for each analyte of interest. Thus, the detection zone includes a first capture zone including a first immobilized capture agent that specifically binds to CRP, a second capture zone including a second immobilized capture agent that specifically binds to Analyte 2, and a third capture zone including a third immobilized capture agent that specifically binds to Analyte 3.

In this example, anti-CRP antibody was deposited at the first capture zone in an amount of 2.4 mg/mL at 0.75 µL/cm, anti-Analyte2 antibody was deposited at the second capture zone in an amount of 2.4 mg/mL at 0.75 µL/cm, and anti-Analyte3 antibody was deposited at the third capture zone in an amount of 3 mg/mL at 0.75 µL/cm.

In this example, the detection zone also includes a positive control capture zone and a negative control capture zone. The positive control capture zone is prepared to ensure that the assay functions properly. In this example, the positive control capture zone includes immobilized bovine serum albumin derivatized with biotin (BSA-biotin). The immobilized BSA-biotin captures labeled anti-biotin antibody present on the test strip that rehydrate with the fluid sample and flow to the positive control capture zone, indicating proper function of the assay. The labeled anti-biotin antibody is captured at the positive control line, and a positive control signal indicates proper function of the assay. The positive control signal may also be used as a reference line for determining relative signal intensities of the first capture zone, the second capture zone, and the third capture zone to increase accuracy of concentrations of analytes of interest.

The negative control capture zone includes immobilized antibody against interfering components that may be present in the fluid sample. Such interfering components may interfere with the first capture zone, the second capture zone, or the third capture zone, thereby causing an incorrect signal intensity. The interfering components will also bind to the negative control capture zone. Embodiments of readers and data analyzers disclosed herein can process the signal measurements obtained from the negative control zone to correct the signal measured at the first capture zone, the second capture zone, and the third capture zone or to alert an operator that the test was invalid.

### Example 2

### Quantification of CRP, Analyte 2, or Analyte 3 using a Single Multiplex Lateral Flow Assay

Due to the significantly varied concentrations of CRP compared to Analyte 2 (such as but not limited to PCT) and Analyte 3 (such as but not limited to Mx1), sandwich-type lateral flow assays are generally unsuitable to quantify CRP when present at high concentrations and simultaneously quantify the concentration of Analyte 2 and Analyte 3 when present (at either low or high concentrations). When present in a sample of typical volume at any concentration, Analyte 2 and Analyte 3 are present on the order of 1-999 ng/mL or 1-999 pg/mL, in contrast to CRP, which, when present in a sample of the same typical volume, is present in concentrations on the order of 1-999 µg/mL.Determining elevated concentrations of CRP previously required serial dilutions of the sample, resulting in an inefficient and laborious process, and also causing a decrease in concentration of the already low concentration of Analyte 2 and Analyte 3, to concentrations that would not be detectable. Using lateral flow devices, test systems, and methods described herein, however, high concentrations of CRP and significantly lower concentrations of Analyte 2 and Analyte 3 (for example, one-millionth the concentration of the CRP) can be accurately, reliably, and quickly quantified.

Lateral flow assays as prepared in Example 1 were contacted with a sample including various concentrations of CRP, Analyte 2, or Analyte 3, as described in Table 1 below. Fluid samples were prepared by adding the amounts of CRP, Analyte 2, or Analyte 3 shown in Table 1 in 45 µL of human serum. The sample was received on the lateral flow assay, and after 30 seconds, chased with 45 µL of HEPES buffer. After ten minutes, the optical signal was measured. Figures 7A-7C illustrate the resulting dose response curves for the lateral flow assay. Figure 7A shows a dose response curve for increasing concentrations of CRP, with no Analyte 2 or Analyte 3 present. In Figure 7A, the signal intensity of the dose response curve for CRP (plotted with squares) decreases with increasing concentration of CRP, consistent with competition of unlabeled CRP present in the sample with the antibody-label-CRP complex. In Figure 7A, the signal intensities for the dose response curves for Analyte 2 (plotted with triangles) and Analyte 3 (plotted with circles) remains at or near zero, indicating an absence of Analyte 2 and Analyte 3 in the sample (or presence of Analyte 2 and Analyte 3 at a level below the detectable level).

Figure 7B shows a dose response curve for increasing concentrations of Analyte 2, with no CRP or Analyte 3 present. In Figure 7B, the signal intensity of the dose response curve for Analyte 2 (triangles) increases with increasing concentration of Analyte 2. In Figure 7B, the signal intensity for the dose response curve for Analyte 3 (circles) remains at or near zero, indicating an absence of Analyte 3 (or presence of Analyte 3 at a level that is below the detectable level) in the sample. Furthermore, the signal intensity for the dose response curve for CRP (squares) remains at a signal maximum (near 70 AU), indicating an absence of CRP (or presence of CRP at a level that is below the detectable level) in the sample.

Figure 7C shows a dose response curve for increasing concentrations of Analyte 3, with no CRP or Analyte 2 present. In Figure 7C, the signal intensity of the dose response curve for Analyte 3 (circles) increases with increasing concentration of Analyte 3. In Figure 7C, the signal intensity for the dose response curve for Analyte 2 (triangles) remains at or near zero, indicating an absence of Analyte 2 in the sample (or presence of Analyte 2 at a level that is below the detectable level). Furthermore, the signal intensity for the dose response curve for CRP (squares) remains at a signal maximum (near 70 AU), indicating an absence of CRP (or presence of CRP at a level that is below the detectable level) in the sample.

**Table 1: Lateral Flow Assay for CRP, Analyte 2, and Analyte 3**

| [CRP] (µg/mL) in Serum Sample | [Analyte 2] (pg/mL) in Serum Sample | [Analyte 3] (pg/mL) in Serum Sample | Signal (AU) at First Capture Zone | Signal (AU) at Second Capture Zone | Signal (AU) at Third Capture Zone |
|---|---|---|---|---|---|
| 0 | 0 | 0 | 70.5 | 1.8 | 0.91 |
| 5 | 0 | 0 | 63.3 | 1.8 | 0.69 |
| 10 | 0 | 0 | 54.6 | 1.8 | 0.56 |
| 20 | 0 | 0 | 39.8 | 1.9 | 0.69 |
| 40 | 0 | 0 | 24.8 | 1.8 | 0.54 |
| 60 | 0 | 0 | 16.5 | 2.1 | 0.87 |
| 100 | 0 | 0 | 10.0 | 2.1 | 0.53 |
| 150 | 0 | 0 | 6.4 | 2.1 | 0.48 |
| 0 | 0 | 0 | 71.57 | 0.03 | 0.59 |
| 0 | 62.5 | 0 | 72.04 | 0.64 | 0.37 |
| 0 | 125 | 0 | 72.24 | 1.71 | 0.30 |
| 0 | 250 | 0 | 71.97 | 4.48 | 0.34 |
| 0 | 500 | 0 | 71.40 | 9.56 | 0.15 |
| 0 | 1000 | 0 | 72.45 | 18.51 | 0.25 |
| 0 | 0 | 0 | 71.57 | 0.03 | 0.59 |
| 0 | 0 | 31.25 | 71.09 | 0.00 | 1.45 |
| 0 | 0 | 62.5 | 71.65 | 0.00 | 3.00 |
| 0 | 0 | 125 | 70.98 | 0.00 | 5.64 |
| 0 | 0 | 250 | 71.69 | 0.00 | 10.62 |
| 0 | 0 | 500 | 71.51 | 0.00 | 19.72 |

### Example 3

### Simultaneous Quantification of CRP, Analyte 2, and Analyte 3 Using a Single Multiplex Lateral Flow Assay

Example 2 demonstrates a single multiplex lateral flow assay for simultaneously detecting CRP, Analyte 2, or Analyte 3 in a serum sample. This example further demonstrates a single lateral flow assay for detecting the presence of a combination of any one or more of CRP, Analyte 2, and Analyte 3 in a serum sample.

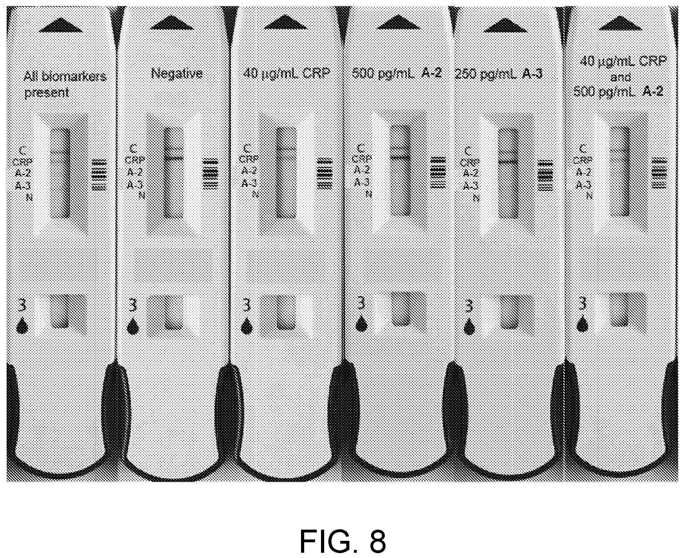

Lateral flow assays as prepared in Example 1 were contacted with a sample including combinations of CRP, Analyte 2, and Analyte 3, as described in Table 2 below. Fluid samples were prepared by adding either CRP in an amount of 40 µg/mL, Analyte 2 in an amount of 500 pg/mL, or Analyte 3 in an amount of 250 pg/mL, or combinations thereof, as shown in Table 2 in 45 µL of human serum substitute. The sample was received on the lateral flow assay, and after 30 seconds, chased with 45 µL of HEPES buffer. After ten minutes, the optical signal was observed. Figures 8 illustrates the lateral flow assay devices for each condition in Table 2. Figure 8 shows six lateral flow assay devices under the following conditions (from left to right): the presence of each of CRP, Analyte 2, and Analyte 3 (see also Figures 1A and 1B); the absence of CRP, Analyte 2, and Analyte 3 (see also Figures 2A and 2B); the presence of CRP alone (see also Figures 3A and 3B); the presence of Analyte 2 alone (see also Figures 4A and 4B); the presence of Analyte 3 alone (see also Figures 5A and 5B); and the presence of both CRP and Analyte 2 (see also Figures 6A and 6B). In Figure 8, lateral flow assays that do not have CRP present in the sample result in a maximum signal intensity at the CRP capture zone, whereas lateral flow assays where CRP was present in the sample result in decreased signal intensity at the CRP capture zone. Conversely, the presence of Analyte 2 and Analyte 3 increases signal intensity at the Analyte 2 capture zone or Analyte 3 capture zone, respectively. Samples having a combination of CRP, Analyte 2, and Analyte 3 indicate the presence of the respective analyte, and may be used for a determination of inflammation, a viral infection, or a bacterial infection.

**Table 2: Lateral Flow Assay for Testing Combination of CRP, Analyte 2, and Analyte 3**

| Fluid Sample Analytes | First Capture Zone | Second Capture Zone | Third Capture Zone | Indication |
|---|---|---|---|---|
| CRP, Analyte 2 and Analyte 3 | Moderate decreased signal | Increased signal | Increased signal | Viral infection |
| None | Maximum signal | No signal | No signal | No analyte present |
| CRP | Decreased signal | No signal | No signal | inflammation |
| Analyte 2 | Maximum signal | Increased Signal | No signal | Analyte 2 present |
| Analyte 3 | Maximum signal | No signal | Increased signal | Analyte 3 present |
| CRP and Analyte 2 | Decreased signal | Increased signal | No signal | Bacterial infection |

Examples 2 and 3 demonstrate the efficacy of an example lateral flow assay as described herein for determining the concentration of a plurality of analytes of interest when one or more analytes of interest are present in a high concentration and one or more analytes of interest are present in a low concentration, even when the concentration of the one or more analytes of interest present in a high concentration is present in an amount of millions of times greater than the amount of analytes of interest in a low concentration. Examples 2 and 3 employ two sandwich-type lateral flow assays for determining two analytes in a low concentration in combination with a sandwich-type assay configured to detect an analyte in a high concentration on a single test strip, but it will be understood that the present disclosure is applicable other configurations. As another non-limiting example, the lateral flow assays described herein can employ one sandwich-type lateral flow assay for determine one analyte in a low concentration in combination with two sandwich-type assays configured to detect two analytes in a high concentration on a single test strip.

Advantageously, the lateral flow assay according to the present disclosure allows the concentration of CRP to be accurately determined at concentrations greater than 10 µg/mL and simultaneously allows the concentration of Analyte 2 and Analyte 3 to be accurately determined at concentrations as low as 30 and 1000 pg/mL. This is particularly advantageous in accurately diagnosing disease and non-disease conditions, wherein one or more of CRP, Analyte 2, and Analyte 3 may be present, such as in an inflammation condition, a viral infection condition, or a bacterial infection condition. The lateral flow assay according to the present disclosure may distinguish between inflammation, a viral infection, or a bacterial infection by determining the concentration of each of CRP, Analyte 2, and Analyte 3 in a single assay. The CRP, Analyte 2, and Analyte 3 can be present in a single sample that is applied to the single assay in a single test event.

Furthermore, lateral flow devices described herein quantify elevated concentrations of a plurality of analytes in a sample in one single assay, without the need to dilute the sample. Assays for determining high concentration of analyte often dilute the sample to decrease total analyte on the assay. Dilution requires additional physical steps as well as further calculations. In addition, although dilution may be helpful for analytes at high concentration, analytes at low concentration suffer from dilution by decreasing the ability to detect low concentration analytes. Thus, dilution is not suitable for a single assay for detecting both low and high concentration analytes. The lateral flow assay of the present disclosure is capable of determining minute differences in a plurality of analyte concentrations based on a signal obtained at the detection zone after a single test.

### Methods of Diagnosing a Condition Using Lateral Flow Assays According to the Present Disclosure

Some embodiments provided herein relate to methods of using lateral flow assays to diagnose a medical condition. In some embodiments, the method includes providing a lateral flow assay as described herein. In some embodiments, the method includes receiving a sample at a sample reservoir of the lateral flow assay.

In some embodiments, the sample is obtained from a source, including an environmental or biological source. In some embodiments, the sample is suspected of having one or more analytes of interest. In some embodiments, the sample is not suspected of having any analytes of interest. In some embodiments, a sample is obtained and analyzed for verification of the absence or presence of a plurality of analytes. In some embodiments, a sample is obtained and analyzed for the quantity of a plurality of analyte in the sample. In some embodiments, the quantity of any one of the one or more analytes present in a sample is less than a normal value present in healthy subjects, at or around a normal value present in healthy subjects, or above a normal value present in healthy subjects.

In some embodiments, receiving a sample at the sample reservoir of the lateral flow assay includes contacting a sample with a lateral flow assay. A sample may contact a lateral flow assay by introducing a sample to a sample reservoir by external application, as with a dropper or other applicator. In some embodiments, a sample reservoir may be directly immersed in the sample, such as when a test strip is dipped into a container holding a sample. In some embodiments, a sample may be poured, dripped, sprayed, placed, or otherwise contacted with the sample reservoir.

A complex in embodiments of the present disclosure includes an antibody that specifically binds an analyte of interest, a label, and the analyte of interest and can be deposited on a conjugate pad (or label zone) within or downstream of the sample reservoir. The device may include a first complex having an antibody that specifically binds a first analyte of interest, a label, and the first analyte of interest. The complex is used for determination of the presence and/or quantity of analyte that may be present in the sample in high concentrations. Thus, additional complexes may also be included on the device, where the operator is interested in determining the presence and/or quantity of more than one analyte of interest present at high concentration.

The device may further include a labeled antibody includes an antibody that specifically binds an analyte of interest and a label, but does not include the antibody of interest. The device may include a second labeled antibody that includes a second antibody that specifically binds a second analyte of interest and a label, and the device may also include a third labeled antibody that includes a third antibody that specifically binds a third analyte of interest and a label. The labeled antibody is used for determination of the presence and/or quantity of analyte that may be present in the sample in low concentrations. Thus, additional labeled antibodies may also be included on the device, where the operator is interested in determining the presence and/or quantity of more the second analyte of interest and the third analyte of interest. The labeled antibody can be deposited on a conjugate pad (or label zone) within or downstream of the sample reservoir.

The first complex, the second labeled antibody, and the third labeled antibody can be integrated on the conjugate pad by physical or chemical bonds. The sample solubilizes the first complex, the second labeled antibody, and the third labeled antibody after the sample is added to the sample reservoir, releasing the bonds holding the first complex, the second labeled antibody, and the third labeled antibody to the conjugate pad. The second labeled antibody binds to the second analyte of interest, if present in the sample, forming a second complex. The third labeled antibody binds to the third analyte of interest, if present in the sample, forming a third complex. The sample, including first analyte of interest, or no first analyte of interest, the first complex, the second complex (when second analyte of interest is present in the sample), and the third complex (when third analyte of interest is present in the sample) flow along the fluid front through the lateral flow assay to a detection zone. The detection zone may include a capture zone for capturing each complex. For example, the detection zone may include a first capture zone for capturing a first complex, a second capture zone for capturing a second complex, and a third capture zone for capturing a third complex. A first capture agent immobilized at the first capture zone binds first analyte (if present) and the first complex. When first complex binds to first capture agent at the first capture zone, a first signal from the label is detected. The first signal may include an optical signal as described herein. When low concentrations of first analyte are present in the sample (such as levels at or below healthy levels), a maximum intensity signal at the first capture zone is detected. At elevated concentrations of first analyte (such as levels above healthy values), the intensity of the first signal decreases in an amount proportionate to the amount of first analyte in the sample. The first signal is compared to values on a dose response curve for the first analyte of interest, and the concentration of first analyte in the sample is determined.

A second capture agent immobilized at the second capture zone binds the second complex. When second complex binds to the second capture agent at the second capture zone, a second signal from the label is detected. The second signal may include an optical signal as described herein and may be the same wavelength as the first signal, or may be a different wavelength from the first signal. As concentration of the second analyte increase, the formation of second complex increases, resulting in increasing amounts of captured second complex by the second capture agent at the second capture zone, which results in increased second signal intensity.

A third capture agent immobilized at the third capture zone binds the third complex. When third complex binds to the third capture agent at the third capture zone, a third signal from the label is detected. The third signal may include an optical signal as described herein and may be the same wavelength as the first signal or the second signal, or may be a different wavelength from the first signal or the second signal. As concentration of the third analyte increase, the formation of third complex increases, resulting in increasing amounts of captured third complex by the third capture agent at the third capture zone, which results in increased third signal intensity.

In some embodiments, the first analyte is present in elevated concentrations. Elevated concentrations of first analyte can refer to a concentration of first analyte that is above healthy levels. Thus, elevated concentration of first analyte can include a concentration of first analyte that is 5%, 10%, 15%, 20%, 25%, 30%, 35%, 40%, 50%, 60%, 70%, 80%, 90%, 100%, 125%, 150%, 200%, or greater than a healthy level. In some embodiments, a first analyte of interest includes C-reactive protein (CRP), which is present in blood serum of healthy individuals in an amount of about 1 to about 10 µg/mL. Thus, elevated concentrations of CRP in a sample includes an amount of 15, 20, 25, 30, 35, 40, 45, 50, 55, 60, 65, 70, 75, 80, 85, 90, 95, 100, 110, 120, 130, 140, 150, 160, 170, 180, 190, or 200 µg/mL or greater.

In some embodiments, the second analyte is present in elevated concentrations. Elevated concentrations of second analyte can refer to a concentration of second analyte that is above healthy levels. Thus, elevated concentration of second analyte can include a concentration of second analyte that is 5%, 10%, 15%, 20%, 25%, 30%, 35%, 40%, 50%, 60%, 70%, 80%, 90%, 100%, 125%, 150%, 200%, or greater than a healthy level. In some embodiments, a second analyte of interest includes procalcitonin (PCT), which is present in blood serum of healthy individuals in an amount less than about 0.05 ng/mL. Thus, elevated concentrations of PCT in a sample includes an amount of 0.05 ng/mL or greater. PCT present in a sample in about of about 0.5 to 2.0 ng/mL may be very elevated, and potentially indicative of sepsis.

In some embodiments, the third analyte is present in elevated concentrations. Elevated concentrations of third analyte can refer to a concentration of third analyte that is above healthy levels. Thus, elevated concentration of third analyte can include a concentration of third analyte that is 5%, 10%, 15%, 20%, 25%, 30%, 35%, 40%, 50%, 60%, 70%, 80%, 90%, 100%, 125%, 150%, 200%, or greater than a healthy level. In some embodiments, a third analyte of interest includes Mx1, which may be present in blood serum of healthy individuals in an amount and is present in elevated concentrations greater than the amount when a viral infection is present.

In some embodiments, upon determination that a first analyte, a second, analyte, or a third analyte, or a combination thereof is present in a sample in elevated concentrations, the subject is diagnosed with a certain disease. For example, elevated CRP concentrations, but no increase in Analyte 2 or Analyte 3, can be indicative of inflammation. Elevated Analyte 2 and CRP concentrations, but no increase in Analyte 3, can be indicative of a bacterial infection. Elevated concentrations of all of CRP, Analyte 2, and Analyte 3 can be indicative of a viral infection. In some embodiments, diagnosis of inflammation is made when the concentration of CRP is deteremined to be 15, 20, 25, 30, 35, 40, 45, 50, 55, 60, 65, 70, 75, 80, 85, 90, 95, 100, 110, 120, 130, 140, 150, 160, 170, 180, 190, or 200 µg/mL or greater, but the concentrations of both Analyte 2 and Analyte 3 are determined to be within healthy range. In some embodiments, diagnosis of a bacterial infection is made when the concentration of CRP is determined to be 15, 20, 25, 30, 35, 40, 45, 50, 55, 60, 65, 70, 75, 80, 85, 90, 95, 100, 110, 120, 130, 140, 150, 160, 170, 180, 190, or 200 µg/mL or greater and the concentration of Analyte 2 is determined to be around or greater than 0.05 ng/mL, but the concentration of Analyte 3 is determined to be within healthy range. In some embodiments, diagnosis of a viral infection is made when the concentration of CRP is present at low concentrations and both Analyte 2 and Analyte 3 concentrations are elevated. In non-limiting examples, diagnosis of a viral infection is made when the concentration of CRP is determined to be not elevated (for example betwen about 1 µg/mL and about 10 µg/mL), the concentration of Analyte 2 is not elevanted, and the concentration of Analyte 3 is elevated.

The diagnosis of a condition, including inflammation, a bacterial infection, or a viral infection, can be made from a single application of a single sample on a single lateral flow assay device described herein, even where the concentration of one analyte of interest (such as CRP) is present in an amount significantly greater than another analyte of interest (such as Analyte 2 and Analyte 3). Thus, a single device is capable of accurately determining the presence and/or concentration of an analyte of interest present in an amount of 10 million, 9 million, 8 million, 7 million, 6 million, 5 million, 4 million, 3 million, 2 million, 1 million, 500,000, 100,000, 50,000, 10,000, 5,000, 1,000, 500, 100, or 10 times greater than an analyte present at low concentration.

The above-described example implementations of lateral flow devices, test systems, and method according to the present disclosure detect the presence and/or the concentration of CRP, Analyte 2, and Analyte 3 in a single sample applied to a single lateral flow assay (such as a single lateral flow assay test strip) in a single application. It will be understood that the present disclosure is not limited to these example implementations. For example, in another non-limiting example, the lateral flow devices, test systems, and method according to the present disclosure can detect the presence and/or the concentration of CRP, any analyte indicative of bacterial and viral infection, and any analyte indicative of viral infection in a single sample applied to a single lateral flow assay (such as a single lateral flow assay test strip) in a single application. In one non-limiting example, the lateral flow devices, test systems, and method according to the present disclosure can detect the presence and/or the concentration of CRP, TRAIL, IP-10, Mx1, and PCT (or in any combination of these) in a single sample applied to a single lateral flow assay (such as a single lateral flow assay test strip) in a single application. In yet a further non-limiting example, the lateral flow devices, test systems, and method according to the present disclosure can detect the presence and/or the concentration of CRP and any of TRAIL, IP-10, Mx1, and PCT in a single sample applied to a single lateral flow assay (such as a single lateral flow assay test strip) in a single application. It will be understood that the particular analytes listed in these non-limiting examples are to illustrate, rather than limit, the present disclosure; any analyte of interest can be detected and measured using the lateral flow devices, test systems, and methods described herein.

### Additional Implementations of Multiplex Lateral Flow Assays According to the Present Disclosure that Can Detect the Presence and Concentration of High Concentration Analytes

Lateral flow devices, test systems, and methods according to the present disclosure precisely determine the presence or quantity of a plurality of analytes of interest in situations where one or more analytes of interest are present in the sample at an elevated or high concentration and one or more analytes of interest are present in the sample at a low concentration. Advantageously, lateral flow devices, test systems, and methods described herein determine the presence or quantity of analytes of interest present in a single sample at significantly different concentrations after applying the single sample to one lateral flow assay, such as a single test strip, in a single test event. Lateral flow assays described herein are thus capable of detecting a plurality of analytes simultaneously, in a single sample, even when analytes are present in significantly different concentration ranges. Example lateral flow devices, test systems, and methods that determine the presence or quantity of one or more analytes of interest present in the sample at a high concentration were described above with reference to non-limiting embodiments illustrated in Figures 1A-6B. Additional example implementations are described in International Application No. PCT/US2018/039347, filed June 25, 2018.

Multiplex lateral flow devices, test systems, and methods of the present disclosure can determine the presence or quantity of one or more analytes of interest present in the sample at a high concentration using additional techniques. For example, additional lateral flow devices, test systems, and methods described in International Application No. PCT/US2018/063586, filed December 3, 2018 can be implemented in multiplex lateral flow devices, test systems, and methods according to the present disclosure to determine the presence or quantity of one or more analytes of interest present in the sample at a high concentration.

Implementations described in International Application No. PCT/US2018/063586 relate to an assay test strip including a flow path configured to receive a fluid sample; a sample receiving zone coupled to the flow path; a capture zone; a labeled antibody or fragment thereof; and oversized particles in the flow path upstream of the capture zone. The capture zone is coupled to the flow path downstream of the sample receiving zone and including an immobilized capture agent specific to an analyte of interest (such as but not limited to CRP). The labeled antibody or fragment thereof is coupled to the flow path upstream of the capture zone specific to the analyte of interest. The oversized particles are conjugated to an antibody or fragment thereof specific to the analyte of interest to form antibody-conjugated oversized particles of a size and dimension to remain upstream of the capture zone when the fluid sample is received on the assay test strip. The flow path in this example implementation is configured to receive a fluid sample including the analyte of interest (such as but not limited to CRP). The labeled antibody or fragment thereof and the antibody-conjugated oversized particles compete to specifically bind the analyte of interest. The labeled antibody or fragment thereof is configured to flow with bound analyte of interest in the flow path to the capture zone when the fluid sample is received on the assay test strip. The labeled antibody bound to the analyte of interest is captured at the capture zone and emits a detectable signal.

In some instances, the flow path is configured to receive a fluid sample that does or does not include analyte of interest (such as but not limited to CRP). The antibody-conjugated oversized particles specifically bind to a known quantity of analyte of interest, thereby retaining a known quantity of analyte of interest upstream of the capture zone.

The assay test strip in this example includes a control zone downstream of the capture zone. The control zone includes antibody that specifically binds to the labeled antibody or fragment thereof that does not bind to analyte of interest and flows past the capture zone. When the fluid sample does not include an analyte of interest, the labeled antibody or fragment thereof flows to the control zone and emits an optical signal at the control zone only, indicating absence of the analyte of interest in the fluid sample. The immobilized capture agent includes an antibody or a fragment thereof specific to the analyte of interest. In some embodiments, the antibody-conjugated oversized particles are integrated onto a surface of the test strip. In some embodiments, the oversized particles include gold particles, latex beads, magnetic beads, or silicon beads. In some embodiments, the oversized particle is about 1 µm to about 15 µm in diameter. In some embodiments, the fluid sample is selected from the group consisting of a whole blood, venous blood, capillary blood, plasma, serum, urine, sweat, or saliva sample. In some embodiments, the analyte of interest includes C-reactive protein (CRP) and the antibody or fragment thereof conjugated to the oversized particle includes an anti-CRP antibody or fragment thereof bound to the CRP.

The above-described implementation to measure the presence and concentration of a high concentration analyte of interest, such as but not limited to CRP, can be included on a single multiplex lateral flow assay test strip according to the present disclosure to detect a plurality of analytes of interest that are present in a sample at significantly different concentrations. For example, embodiments of the lateral flow devices, test systems, and methods according to the present disclosure can employ, on a single test strip, two sandwich-type lateral flow assays for determining two analytes in a low concentration in a single sample (such as, for example, a second analyte of interest 113 and a third analyte of interest 114 described above with reference to Figures 4A-4B, 5A-5B, and Examples 2 and 3) in combination with a sandwich-type assay described in International Application No. PCT/US2018/063586 that is configured to detect an analyte of interest in a high concentration (such as but not limited to CRP) in the same single sample applied to the single test strip in a single test event.

### Example Test Systems Including Lateral Flow Assays According to the Present Disclosure

Lateral flow assay test systems described herein can include a lateral flow assay test device (such as but not limited to a test strip), a housing including a port configured to receive all or a portion of the test device, a reader including a light source and a light detector, a data analyzer, and combinations thereof. A housing may be made of any one of a wide variety of materials, including plastic, metal, or composite materials. The housing forms a protective enclosure for components of the diagnostic test system. The housing also defines a receptacle that mechanically registers the test strip with respect to the reader. The receptacle may be designed to receive any one of a wide variety of different types of test strips. In some embodiments, the housing is a portable device that allows for the ability to perform a lateral flow assay in a variety of environments, including on the bench, in the field, in the home, or in a facility for domestic, commercial, or environmental applications.

A reader may include one or more optoelectronic components for optically inspecting the exposed areas of the detection zone of the test strip, and capable of detecting multiple capture zones within the detection zone. In some implementations, the reader includes at least one light source and at least one light detector. In some embodiments, the light source may include a semiconductor light-emitting diode and the light detector may include a semiconductor photodiode. Depending on the nature of the label that is used by the test strip, the light source may be designed to emit light within a particular wavelength range or light with a particular polarization. For example, if the label is a fluorescent label, such as a quantum dot, the light source would be designed to illuminate the exposed areas of the capture zone of the test strip with light in a wavelength range that induces fluorescent emission from the label. Similarly, the light detector may be designed to selectively capture light from the exposed areas of the capture zone. For example, if the label is a fluorescent label, the light detector would be designed to selectively capture light within the wavelength range of the fluorescent light emitted by the label or with light of a particular polarization. On the other hand, if the label is a reflective-type label, the light detector would be designed to selectively capture light within the wavelength range of the light emitted by the light source. To these ends, the light detector may include one or more optical filters that define the wavelength ranges or polarizations axes of the captured light. A signal from a label can be analyzed, using visual observation or a spectrophotometer to detect color from a chromogenic substrate; a radiation counter to detect radiation, such as a gamma counter for detection of ¹²⁵I; or a fluorometer to detect fluorescence in the presence of light of a certain wavelength. Where an enzyme-linked assay is used, quantitative analysis of the amount of an analyte of interest can be performed using a spectrophotometer. Lateral flow assays described herein can be automated or performed robotically, if desired, and the signal from multiple samples can be detected simultaneously. Furthermore, multiple signals can be detected in for plurality of analytes of interest, including when the label for each analyte of interest is the same or different.

The data analyzer processes the signal measurements that are obtained by the reader. In general, the data analyzer may be implemented in any computing or processing environment, including in digital electronic circuitry or in computer hardware, firmware, or software. In some embodiments, the data analyzer includes a processor (e.g., a microcontroller, a microprocessor, or ASIC) and an analog-to-digital converter. The data analyzer can be incorporated within the housing of the diagnostic test system. In other embodiments, the data analyzer is located in a separate device, such as a computer, that may communicate with the diagnostic test system over a wired or wireless connection. The data analyzer may also include circuits for transfer of results via a wireless connection to an external source for data analysis or for reviewing the results.

In general, the results indicator may include any one of a wide variety of different mechanisms for indicating one or more results of an assay test. In some implementations, the results indicator includes one or more lights (e.g., light-emitting diodes) that are activated to indicate, for example, the completion of the assay test. In other implementations, the results indicator includes an alphanumeric display (e.g., a two or three character light-emitting diode array) for presenting assay test results.

Test systems described herein can include a power supply that supplies power to the active components of the diagnostic test system, including the reader, the data analyzer, and the results indicator. The power supply may be implemented by, for example, a replaceable battery or a rechargeable battery. In other embodiments, the diagnostic test system may be powered by an external host device (e.g., a computer connected by a USB cable).

### Features of Example Lateral Flow Devices

Lateral flow devices described herein can include a sample reservoir (also referred to as a sample receiving zone) where a fluid sample is introduced to a test strip, such as but not limited to an immunochromatographic test strip present in a lateral flow device. In one example, the sample may be introduced to sample reservoir by external application, as with a dropper or other applicator. The sample may be poured or expressed onto the sample reservoir. In another example, the sample reservoir may be directly immersed in the sample, such as when a test strip is dipped into a container holding a sample.

Lateral flow devices described herein can include a solid support or substrate. Suitable solid supports include but are not limited to nitrocellulose, the walls of wells of a reaction tray, multi-well plates, test tubes, polystyrene beads, magnetic beads, membranes, and microparticles (such as latex particles). Any suitable porous material with sufficient porosity to allow access by labeled agents and a suitable surface affinity to immobilize capture agents can be used in lateral flow devices described herein. For example, the porous structure of nitrocellulose has excellent absorption and adsorption qualities for a wide variety of reagents, for instance, capture agents. Nylon possesses similar characteristics and is also suitable. Microporous structures are useful, as are materials with gel structure in the hydrated state.

Further examples of useful solid supports include: natural polymeric carbohydrates and their synthetically modified, cross-linked or substituted derivatives, such as agar, agarose, cross-linked alginic acid, substituted and cross-linked guar gums, cellulose esters, especially with nitric acid and carboxylic acids, mixed cellulose esters, and cellulose ethers; natural polymers containing nitrogen, such as proteins and derivatives, including cross-linked or modified gelatins; natural hydrocarbon polymers, such as latex and rubber; synthetic polymers which may be prepared with suitably porous structures, such as vinyl polymers, including polyethylene, polypropylene, polystyrene, polyvinyl chloride, polyvinylacetate and its partially hydrolyzed derivatives, polyacrylamides, polymethacrylates, copolymers and terpolymers of the above polycondensates, such as polyesters, polyamides, and other polymers, such as polyurethanes or polyepoxides; porous inorganic materials such as sulfates or carbonates of alkaline earth metals and magnesium, including barium sulfate, calcium sulfate, calcium carbonate, silicates of alkali and alkaline earth metals, aluminum and magnesium; and aluminum or silicon oxides or hydrates, such as clays, alumina, talc, kaolin, zeolite, silica gel, or glass (these materials may be used as filters with the above polymeric materials); and mixtures or copolymers of the above classes, such as graft copolymers obtained by initializing polymerization of synthetic polymers on a pre-existing natural polymer.

Lateral flow devices described herein can include porous solid supports, such as nitrocellulose, in the form of sheets or strips. The thickness of such sheets or strips may vary within wide limits, for example, from about 0.01 to 0.5 mm, from about 0.02 to 0.45 mm, from about 0.05 to 0.3 mm, from about 0.075 to 0.25 mm, from about 0.1 to 0.2 mm, or from about 0.11 to 0.15 mm. The pore size of such sheets or strips may similarly vary within wide limits, for example from about 0.025 to 15 microns, or more specifically from about 0.1 to 3 microns; however, pore size is not intended to be a limiting factor in selection of the solid support. The flow rate of a solid support, where applicable, can also vary within wide limits, for example from about 12.5 to 90 sec/cm (i.e., 50 to 300 sec/4 cm), about 22.5 to 62.5 sec/cm (i.e., 90 to 250 sec/4 cm), about 25 to 62.5 sec/cm (i.e., 100 to 250 sec/4 cm), about 37.5 to 62.5 sec/cm (i.e., 150 to 250 sec/4 cm), or about 50 to 62.5 sec/cm (i.e., 200 to 250 sec/4 cm). In specific embodiments of devices described herein, the flow rate is about 35 sec/cm (i.e., 140 sec/4 cm). In other specific embodiments of devices described herein, the flow rate is about 37.5 sec/cm (i.e., 150 sec/4 cm).

The surface of a solid support may be activated by chemical processes that cause covalent linkage of an agent (e.g., a capture reagent) to the support. As described below, the solid support can include a conjugate pad. Many other suitable methods may be used for immobilizing an agent (e.g., a capture reagent) to a solid support including, without limitation, ionic interactions, hydrophobic interactions, covalent interactions and the like.

Except as otherwise physically constrained, a solid support may be used in any suitable shapes, such as films, sheets, strips, or plates, or it may be coated onto or bonded or laminated to appropriate inert carriers, such as paper, glass, plastic films, or fabrics.

Lateral flow devices described herein can include a conjugate pad, such as a membrane or other type of material that includes a capture reagent. The conjugate pad can be a cellulose acetate, cellulose nitrate, polyamide, polycarbonate, glass fiber, membrane, polyethersulfone, regenerated cellulose (RC), polytetra-fluorethylene, (PTFE), Polyester (e.g. Polyethylene Terephthalate), Polycarbonate (e.g., 4,4-hydroxy-diphenyl-2,2'-propane), Aluminum Oxide, Mixed Cellulose Ester (e.g., mixture of cellulose acetate and cellulose nitrate), Nylon (e.g., Polyamide, Hexamethylene-diamine, and Nylon 66), Polypropylene, PVDF, High Density Polyethylene (HDPE)+nucleating agent "aluminum dibenzoate" (DBS) (e.g. 80 u 0.024 HDPE DBS (Porex)), and HDPE.

Lateral flow devices described herein are highly sensitive to a plurality of analytes of interest that are present in a sample at significantly different concentrations, such as at high concentrations (in the 10s to 100s of µg/mL) and at low concentrations (in the 1s to 10s of pg/mL). "Sensitivity" refers to the proportion of actual positives which are correctly identified as such (for example, the percentage of infected, latent or symptomatic subjects who are correctly identified as having a condition). Sensitivity may be calculated as the number of true positives divided by the sum of the number of true positives and the number of false negatives.

Lateral flow devices described herein can accurately measure a plurality of analytes of interest in many different kinds of samples. Samples can include a specimen or culture obtained from any source, as well as biological and environmental samples. Biological samples may be obtained from animals (including humans) and encompass fluids, solids, tissues, and gases. Biological samples include urine, saliva, and blood products, such as plasma, serum and the like. Such examples are not however to be construed as limiting the sample types applicable to the present disclosure.

In some embodiments the sample is an environmental sample for detecting a plurality of analytes in the environment. In some embodiments, the sample is a biological sample from a subject. In some embodiments, a biological sample can include peripheral blood, sera, plasma, ascites, urine, cerebrospinal fluid (CSF), sputum, saliva, bone marrow, synovial fluid, aqueous humor, amniotic fluid, cerumen, breast milk, broncheoalveolar lavage fluid, semen (including prostatic fluid), Cowper's fluid or pre-ejaculatory fluid, female ejaculate, sweat, fecal matter, hair, tears, cyst fluid, pleural and peritoneal fluid, pericardial fluid, lymph, chyme, chyle, bile, interstitial fluid, menses, pus, sebum, vomit, vaginal secretions, mucosal secretion, stool water, pancreatic juice, lavage fluids from sinus cavities, bronchopulmonary aspirates, or other lavage fluids.

As used herein, "analyte" generally refers to a substance to be detected. For instance, analytes may include antigenic substances, haptens, antibodies, and combinations thereof. Analytes include, but are not limited to, toxins, organic compounds, proteins, peptides, microorganisms, amino acids, nucleic acids, hormones, steroids, vitamins, drugs (including those administered for therapeutic purposes as well as those administered for illicit purposes), drug intermediaries or byproducts, bacteria, virus particles, and metabolites of or antibodies to any of the above substances. Specific examples of some analytes include ferritin; creatinine kinase MB (CK-MB); human chorionic gonadotropin (hCG); digoxin; phenytoin; phenobarbitol; carbamazepine; vancomycin; gentamycin; theophylline; valproic acid; quinidine; luteinizing hormone (LH); follicle stimulating hormone (FSH); estradiol, progesterone; C-reactive protein (CRP); lipocalins; IgE antibodies; cytokines; TNF-related apoptosis-inducing ligand (TRAIL); vitamin B2 micro-globulin; interferon gamma-induced protein 10 (IP-10); interferon-induced GTP-binding protein (also referred to as myxovirus (influenza virus) resistance 1, MX1, MxA, IFI-78K, IFI78, MX, MX dynamin like GTPase 1); procalcitonin (PCT); glycated hemoglobin (Gly Hb); cortisol; digitoxin; N-acetylprocainamide (NAPA); procainamide; antibodies to rubella, such as rubella-IgG and rubella IgM; antibodies to toxoplasmosis, such as toxoplasmosis IgG (Toxo-IgG) and toxoplasmosis IgM (Toxo-IgM); testosterone; salicylates; acetaminophen; hepatitis B virus surface antigen (HBsAg); antibodies to hepatitis B core antigen, such as anti-hepatitis B core antigen IgG and IgM (Anti-HBC); human immune deficiency virus 1 and 2 (HIV 1 and 2); human T-cell leukemia virus 1 and 2 (HTLV); hepatitis B e antigen (HBeAg); antibodies to hepatitis B e antigen (Anti-HBe); influenza virus; thyroid stimulating hormone (TSH); thyroxine (T4); total triiodothyronine (Total T3); free triiodothyronine (Free T3); carcinoembryoic antigen (CEA); lipoproteins, cholesterol, and triglycerides; and alpha fetoprotein (AFP). Drugs of abuse and controlled substances include, but are not intended to be limited to, amphetamine; methamphetamine; barbiturates, such as amobarbital, secobarbital, pentobarbital, phenobarbital, and barbital; benzodiazepines, such as librium and valium; cannabinoids, such as hashish and marijuana; cocaine; fentanyl; LSD; methaqualone; opiates, such as heroin, morphine, codeine, hydromorphone, hydrocodone, methadone, oxycodone, oxymorphone and opium; phencyclidine; and propoxyhene. Additional analytes may be included for purposes of biological or environmental substances of interest.

The present disclosure relates to lateral flow assay devices, test systems, and methods to determine the presence and concentration of a plurality of analytes in a sample, including when one or more analytes of interest are present at high concentrations and one or more analytes of interest are present at low concentrations. As discussed above, as used herein, "analyte" generally refers to a substance to be detected, for example a protein. Examples of proteins that can be detected by the lateral flow assay devices, test systems, and methods described herein include, without limitation:
TRAIL: TNF-related apoptosis-inducing ligand (also known as Apo2L, Apo-2 ligand and CD253); representative RefSeq DNA sequences are NC_000003.12; NC_018914.2; and NT_005612.17 and representative RefSeq Protein sequence accession numbers are NP_001177871.1; NP_001177872.1; and NP_003801.1. The TRAIL protein belongs to the tumor necrosis factor (TNF) ligand family.
CRP: C-reactive protein; representative RefSeq DNA sequences are NC_000001.11; NT_004487.20; and NC_018912.2 and a representative RefSeq Protein sequence accession numbers is NP_000558.2.
IP-10: Chemokine (C-X-C motif) ligand 10; representative RefSeq DNA sequences are NC_000004.12; NC_018915.2; and NT_016354.20 and a RefSeq Protein sequence is NP_001556.2.
PCT: Procalcitonin is a peptide precursor of the hormone calcitonin. A representative RefSeq amino acid sequence of this protein is NP_000558.2. Representative RefSeq DNA sequences include NC_000001.11, NT_004487.20, and NC_018912.2.
MX1: Interferon-induced GTP-binding protein Mx1 (also known as interferon-induced protein p78, Interferon-regulated resistance GTP-binding protein, MxA). Representative RefSeq amino acid sequences of this protein are NP_001138397.1; NM_001144925.2; NP_001171517.1; and NM_001178046.2.

Lateral flow assay devices, test systems, and methods according to the present disclosure can measure either the soluble and/or the membrane form of the TRAIL protein. In one embodiment, only the soluble form of TRAIL is measured.

Lateral flow devices described herein can include a label. Labels can take many different forms, including a molecule or composition bound or capable of being bound to an analyte, analyte analog, detector reagent, or binding partner that is detectable by spectroscopic, photochemical, biochemical, immunochemical, electrical, optical or chemical means. Examples of labels include enzymes, colloidal gold particles (also referred to as gold nanoparticles), colored latex particles, radioactive isotopes, co-factors, ligands, chemiluminescent or fluorescent agents, protein-adsorbed silver particles, protein-adsorbed iron particles, protein-adsorbed copper particles, protein-adsorbed selenium particles, protein-adsorbed sulfur particles, protein-adsorbed tellurium particles, protein-adsorbed carbon particles, and protein-coupled dye sacs. The attachment of a compound (e.g., a detector reagent) to a label can be through covalent bonds, adsorption processes, hydrophobic and/or electrostatic bonds, as in chelates and the like, or combinations of these bonds and interactions and/or may involve a linking group.

The term "specific binding partner (or binding partner)" refers to a member of a pair of molecules that interacts by means of specific, noncovalent interactions that depend on the three-dimensional structures of the molecules involved. Typical pairs of specific binding partners include antigen/antibody, hapten/antibody, hormone/receptor, nucleic acid strand/complementary nucleic acid strand, substrate/enzyme, inhibitor/enzyme, carbohydrate/lectin, biotin/(strept)avidin, receptor/ligands, and virus/cellular receptor, or various combinations thereof.

As used herein, the terms "immunoglobulin" or "antibody" refer to proteins that bind a specific antigen. Immunoglobulins include, but are not limited to, polyclonal, monoclonal, chimeric, and humanized antibodies, Fab fragments, F(ab')2 fragments, and includes immunoglobulins of the following classes: IgG, IgA, IgM, IgD, IbE, and secreted immunoglobulins (sIg). Immunoglobulins generally comprise two identical heavy chains and two light chains. However, the terms "antibody" and "immunoglobulin" also encompass single chain antibodies and two chain antibodies. For simplicity, through the specification the terms "labeled antibody" or "capture antibody" is used, but the term antibody as used herein refers to the antibody as a whole or any fragment thereof. Thus, it is contemplated that when referring to a labeled antibody that specifically binds analyte of interest, the term refers to a labeled antibody or fragment thereof that specifically binds an analyte of interest. Similarly, when referring to a capture antibody, the term refers to a capture antibody or fragment thereof that specifically binds to the analyte of interest.

Antibodies in lateral flow devices, test systems, and methods according to the present disclosure can include a polyclonal antibody. Polyclonal antibodies for measuring any of the analytes of interest disclosed herein include without limitation antibodies that were produced from sera by active immunization of one or more of the following: Rabbit, Goat, Sheep, Chicken, Duck, Guinea Pig, Mouse, Donkey, Camel, Rat and Horse. Antibodies in lateral flow devices, test systems, and methods according to the present disclosure can include a monoclonal antibody.

Antibodies for measuring TRAIL include monoclonal antibodies and polyclonal antibodies for measuring TRAIL. In some embodiments, a TRAIL antibody binds to soluble TRAIL and/or the extracellular domain of TRAIL, e.g., amino acids 90-281. Examples of monoclonal antibodies for measuring TRAIL include without limitation: Mouse, Monoclonal (55B709-3) IgG; Mouse, Monoclonal (2E5) IgG1; Mouse, Monoclonal (2E05) IgG1; Mouse, Monoclonal (M912292) IgG1 kappa; Mouse, Monoclonal (IIIF6) IgG2b; Mouse, Monoclonal (2E1-1B9) IgG1; Mouse, Monoclonal (RIK-2) IgG1, kappa; Mouse, Monoclonal M181 IgG1; Mouse, Monoclonal VI10E IgG2b; Mouse, Monoclonal MAB375 IgG1; Mouse, Monoclonal MAB687 IgG1; Mouse, Monoclonal HS501 IgG1; Mouse, Monoclonal clone 75411.11 Mouse IgG1; Mouse, Monoclonal T8175-50 IgG; Mouse, Monoclonal 2B2.108 IgG1; Mouse, Monoclonal B-T24 IgG1; Mouse, Monoclonal 55B709.3 IgG1; Mouse, Monoclonal D3 IgG1; Goat, Monoclonal C19 IgG; Rabbit, Monoclonal H257 IgG; Mouse, Monoclonal 500-M49 IgG; Mouse, Monoclonal 05-607 IgG; Mouse, Monoclonal B-T24 IgG1; Rat, Monoclonal (N2B2), IgG2a, kappa; Mouse, Monoclonal (1A7-2B7), IgG1; Mouse, Monoclonal (55B709.3), IgG and Mouse, Monoclonal B-S23* IgG1, Human TR AIL/TNFS F 10 MAb (Clone 75411), Mouse IgG1, Human TRAIL/TNFSF10 MAb (Clone 124723), Mouse IgG1, Human TR AIL/TNFS F 10 MAb (Clone 75402), Mouse IgG1.

Antibodies for measuring TRAIL include antibodies that were developed to target epitopes from the following non-exhaustive list: Mouse myeloma cell line NSO-derived recombinant human TRAIL (Thr95-Gly281 Accession # P50591), Mouse myeloma cell line, NSO-derived recombinant human TRAIL (Thr95-Gly281,with an N-terminal Met and 6-His tag Accession # P50591), E. coli-derived, (Vail 14-Gly281, with and without an N-terminal Met Accession #:Q6IBA9), Human plasma derived TRAIL, Human serum derived TRAIL, recombinant human TRAIL where first amino acid is between position 85 - 151 and the last amino acid is at position 249 - 281.

Antibodies for measuring CRP include monoclonal antibodies for measuring CRP and polyclonal antibodies for measuring CRP. Examples of monoclonal antibodies for measuring CRP include without limitation: Mouse, Monoclonal (108-2A2); Mouse, Monoclonal (108-7G41D2); Mouse, Monoclonal (12D-2C-36), IgG1; Mouse, Monoclonal (1G1), IgG1; Mouse, Monoclonal (5A9), IgG2a kappa; Mouse, Monoclonal (63F4), IgG1; Mouse, Monoclonal (67A1), IgG1; Mouse, Monoclonal (8B-5E), IgG1; Mouse, Monoclonal (B893M), IgG2b, lambda; Mouse, Monoclonal (C1), IgG2b; Mouse, Monoclonal (C11F2), IgG; Mouse, Monoclonal (C2), IgG1; Mouse, Monoclonal (C3), IgG1; Mouse, Monoclonal (C4), IgG1; Mouse, Monoclonal (C5), IgG2a; Mouse, Monoclonal (C6), IgG2a; Mouse, Monoclonal (C7), IgG1; Mouse, Monoclonal (CRP103), IgG2b; Mouse, Monoclonal (CRP11), IgG1; Mouse, Monoclonal (CRP135), IgG1; Mouse, Monoclonal (CRP169), IgG2a; Mouse, Monoclonal (CRP30), IgG1; Mouse, Monoclonal (CRP36), IgG2a; Rabbit, Monoclonal (EPR283Y), IgG; Mouse, Monoclonal (KT39), IgG2b; Mouse, Monoclonal (N-a), IgG1; Mouse, Monoclonal (N1G1), IgG1; Monoclonal (P5A9AT); Mouse, Monoclonal (S5G1), IgG1; Mouse, Monoclonal (SB78c), IgG1; Mouse, Monoclonal (SB78d), IgG1 and Rabbit, Monoclonal (Y284), IgG.

Antibodies for measuring IP-10 include monoclonal antibodies for measuring IP-10 and polyclonal antibodies for measuring IP-10. Examples of monoclonal antibodies for measuring IP-10 include without limitation: IP-10 / CXCL10 Mouse anti-Human Monoclonal (4D5) Antibody (LifeSpan Biosciences), IP-10 / CXCL10 Mouse anti-Human Monoclonal (A00163.01) Antibody (LifeSpan Biosciences), MOUSE ANTI HUMAN IP-10 (AbD Serotec), RABBIT ANTI HUMAN IP-10 (AbD Serotec), IP-10 Human mAb 6D4 (Hycult Biotech), Mouse Anti-Human IP-10 Monoclonal Antibody Clone B-C50 (Diaclone), Mouse Anti-Human IP-10 Monoclonal Antibody Clone B-C55 (Diaclone), Human CXCLlO/IP-10 MAb Clone 33036 (R&D Systems), CXCL10/INP10 Antibody 1E9 (Novus Biologicals), CXCL10/INP10 Antibody 2C1 (Novus Biologicals), CXCL10/INP10 Antibody 6D4 (Novus Biologicals), CXCL10 monoclonal antibody M01A clone 2C1 (Abnova Corporation), CXCL10 monoclonal antibody (M05), clone 1E9 (Abnova Corporation), CXCL10 monoclonal antibody, clone 1 (Abnova Corporation), IP-10 antibody 6D4 (Abeam), IP10 antibody EPR7849 (Abeam), IP10 antibody EPR7850 (Abeam).

Antibodies for measuring IP-10 also include antibodies that were developed to target epitopes from the following non-exhaustive list: Recombinant human CXCLlO/IP-10, non-glycosylated polypeptide chain containing 77 amino acids (aa 22-98) and an N-terminal His tag Interferon gamma inducible protein 10 (125 aa long), IP-10 His Tag Human Recombinant IP-10 produced in E. Coli containing 77 amino acids fragment (22-98) and having a total molecular mass of 8.5 kDa with an amino-terminal hexahistidine tag, E. coli-derived Human IP-10 (Val22-Pro98) with an N-terminal Met, Human plasma derived IP-10, Human serum derived IP-10, recombinant human IP-10 where first amino acid is between position 1-24 and the last amino acid is at position 71-98.

Antibodies for measuring procalcitonin (PCT) include monoclonal antibodies for measuring PCT and polyclonal antibodies for measuring PCT. Monoclonal antibodies for measuring PCT include without limitation: Mouse, Monoclonal IgG1; Mouse, Monoclonal IgG2a; Mouse, Monoclonal IgG2b; Mouse, Monoclonal 44D9 IgG2a; Mouse, Monoclonal 18B7 IgG1; Mouse, Monoclonal G1/G1-G4 IgG1; Mouse, Monoclonal NOD-15 IgG1; Mouse, Monoclonal 22A11 IgG1; Mouse, Monoclonal 42 IgG2a; Mouse, Monoclonal 27A3 IgG2a; Mouse, Monoclonal 14C12 IgG1; Mouse, Monoclonal 24B2 IgGl; Mouse, Monoclonal 38F 11 IgG1; Mouse, Monoclonal 6F10 IgG1.

Antibodies for measuring MxA include monoclonal antibodies for measuring MxA and polyclonal antibodies for measuring MxA. Monoclonal antibodies for measuring MxA include without limitation: Mouse, Monoclonal IgG; Mouse, Monoclonal IgG1; Mouse, Monoclonal IgG2a; Mouse, Monoclonal IgG2b; Mouse, Monoclonal 2G12 IgG1; Mouse, Monoclonal 474CT4-1-5 IgG2b; Mouse, Monoclonal AM39, IgG1; Mouse, Monoclonal 4812 IgG2a; Mouse, Monoclonal 683 IgG2b.

Lateral flow devices according to the present disclosure include a capture agent. A capture agent includes an immobilized agent that is capable of binding to an analyte, including a free (unlabeled) analyte and/or a labeled analyte (such as a first complex, a second complex, or a third complex, as described herein). A capture agent includes an unlabeled specific binding partner that is specific for (i) a labeled analyte of interest, (ii) a labeled analyte or an unlabeled analyte, or for (iii) an ancillary specific binding partner, which itself is specific for the analyte, as in an indirect assay. As used herein, an "ancillary specific binding partner" is a specific binding partner that binds to the specific binding partner of an analyte. For example, an ancillary specific binding partner may include an antibody specific for another antibody, for example, goat anti-human antibody. Lateral flow devices described herein can include a "detection area" or "detection zone" that is an area that includes one or more capture area or capture zone and that is a region where a detectable signal may be detected. Lateral flow devices described herein can include a "capture area" that is a region of the lateral flow device where the capture reagent is immobilized. Lateral flow devices described herein may include more than one capture area. In some cases, a different capture reagent will be immobilized in different capture areas (such as a first capture reagent at a first capture area and a second capture agent at a second capture area). Multiple capture areas may have any orientation with respect to each other on the lateral flow substrate; for example, a first capture area may be distal or proximal to a second (or other) capture area along the path of fluid flow and vice versa. Alternatively, a first capture area and a second (or other) capture area may be aligned along an axis perpendicular to the path of fluid flow such that fluid contacts the capture areas at the same time or about the same time.

Lateral flow devices according to the present disclosure include capture agents that are immobilized such that movement of the capture agent is restricted during normal operation of the lateral flow device. For example, movement of an immobilized capture agent is restricted before and after a fluid sample is applied to the lateral flow device. Immobilization of capture agents can be accomplished by physical means such as barriers, electrostatic interactions, hydrogen-bonding, bioaffinity, covalent interactions or combinations thereof.

Lateral flow devices according to the present disclosure can detect, identify, and in some cases quantify a biologic. A biologic includes chemical or biochemical compounds produced by a living organism which can include a prokaryotic cell line, a eukaryotic cell line, a mammalian cell line, a microbial cell line, an insect cell line, a plant cell line, a mixed cell line, a naturally occurring cell line, or a synthetically engineered cell line. A biologic can include large macromolecules such as proteins, polysaccharides, lipids, and nucleic acids, as well as small molecules such as primary metabolites, secondary metabolites, and natural products.

It is to be understood that the description, specific examples and data, while indicating exemplary embodiments, are given by way of illustration and are not intended to limit the various embodiments of the present disclosure. Various changes and modifications within the present disclosure will become apparent to the skilled artisan from the description and data contained herein, and thus are considered part of the various embodiments of this disclosure.

## Claims

1. A method of detecting a first analyte of interest and a second analyte of interest present in a sample at different concentrations, the method comprising:
providing a lateral flow assay comprising
a first complex coupled to a flow path of the lateral flow assay, the first complex comprising a label, an antibody or a fragment thereof that specifically binds the first analyte, and the first analyte,
a labeled second antibody or fragment thereof coupled to the flow path and configured to specifically bind the second analyte,
a first capture zone downstream of the first complex, the first capture zone comprising a first immobilized capture agent specific to the first analyte, and
a second capture zone downstream of the labeled second antibody or fragment thereof and comprising a second immobilized capture agent specific to the second analyte;
applying the sample to the first complex and the labeled second antibody or fragment thereof;
binding the second analyte to the labeled second antibody or fragment thereof to form a second complex;
flowing the fluid sample and the first complex to the first capture zone, where the first analyte in the fluid sample and the first complex compete to bind to the first immobilized capture agent in the first capture zone;
flowing the second complex in the flow path to the second capture zone and binding the second complex to the second immobilized capture agent in the second capture zone; and
detecting a first signal from the first complex bound to the first immobilized capture agent in the first capture zone and a second signal from the second complex bound to the second immobilized capture agent in the second capture zone.

2. The method of Claim 1, wherein the first analyte of interest is present in the sample at a concentration about six orders of magnitude greater than the concentration of the second analyte of interest present in the sample.

3. The method of Claim 1, wherein the first analyte of interest is present in the sample at a concentration between 1 and 999 µg/ml and the second analyte of interest is present in the sample at a concentration between 1 and 999 pg/ml.

4. The method of Claim 1, wherein the first analyte of interest is present in the sample at a concentration at least one order of magnitude greater than the concentration of the second analyte of interest present in the sample, the order of magnitude comprising one order of magnitude, two orders of magnitude, three orders of magnitude, four orders of magnitude, five orders of magnitude, six orders of magnitude, seven orders of magnitude, eight orders of magnitude, nine orders of magnitude, or ten orders of magnitude.

5. The method of Claim 1, further comprising correlating the first signal to a concentration of the first analyte of interest present in the sample and correlating the second signal to a concentration of the second analyte of interest in the sample.

6. The method of Claim 1, wherein the first signal detected from the first complex bound to the first immobilized capture agent in the first capture zone decreases as the concentration of the first analyte increases in the sample, and wherein the second signal detected from the second complex bound to the second immobilized capture agent in the second capture zone increases as the concentration of the second analyte of interest increases in the sample.

7. The method of Claim 1, further comprising detecting a third analyte of interest in the sample, wherein the lateral flow assay comprises:
a labeled third antibody or fragment thereof coupled to the flow path and configured to specifically bind the third analyte; and
a third capture zone downstream of the labeled third antibody or fragment thereof and comprising a third immobilized capture agent specific to the third analyte optionally, further comprising:
applying the sample to the labeled third antibody or fragment thereof;
binding the third analyte to the labeled third antibody or fragment thereof to form a third complex;
flowing the third complex in the flow path to the third capture zone and binding the third complex to the third immobilized capture agent in the third capture zone; and
detecting a third signal from the third complex bound to the third immobilized capture agent in the third capture zone.

8. The method of Claim 7, further comprising correlating the first signal, the second signal, and the third signal to a concentration of the first analyte, a concentration of the second analyte, and a concentration of the third analyte in the sample, respectively.

9. The method of Claim 8, further comprising indicating a disease condition, a non-disease condition, or no condition based on the respective concentrations of the first analyte, the second analyte, and the third analyte, wherein the disease condition is a viral infection or a bacterial infection, and wherein the non-disease condition is inflammation.

10. The method of Claim 1, wherein the first analyte of interest comprises C-reactive protein, CRP, and the second analyte of interest comprises interferon-induced GTP-binding protein, Mx1.

11. The method of Claim 7, wherein the third analyte of interest comprises procalcitonin, PCT.

12. The method of Claim 1, wherein the sample is a whole blood sample, a venous blood sample, a capillary blood sample, a serum sample, or a plasma sample; or, wherein the sample is not diluted prior to applying the sample to the lateral flow assay.

13. A lateral flow assay configured to detect a first analyte of interest and a second analyte of interest present in a fluid sample at different concentrations, the lateral flow assay comprising:
a first complex coupled to a flow path of the lateral flow assay, the first complex comprising a label, an antibody or a fragment thereof that specifically binds the first analyte, and the first analyte;
a labeled second antibody or fragment thereof coupled to the flow path and configured to specifically bind the second analyte;
a first capture zone downstream of the first complex, the first capture zone comprising a first immobilized capture agent specific to the first analyte; and
a second capture zone downstream of the labeled second antibody or fragment thereof and comprising a second immobilized capture agent specific to the second analyte.

14. The lateral flow assay of Claim 13, wherein the first complex and the labeled second antibody or fragment thereof uncouple from the flow path of the lateral flow assay when the fluid sample is applied to the first complex and the labeled second antibody or fragment thereof, optionally, wherein the labeled second antibody or fragment thereof binds to the second analyte to form a second complex when the fluid sample is applied to the first complex and the labeled second antibody or fragment thereof.

15. The lateral flow assay of Claim 14, wherein, after the fluid sample is applied, the first immobilized capture agent in the first capture zone competitively binds with the first complex and the first analyte that have flowed in the fluid sample to the first capture zone, optionally, wherein, after the fluid sample is applied, the second immobilized capture agent in the second capture zone binds with the second complex that has flowed in the fluid sample to the second capture zone.

16. The lateral flow assay of Claim 15, wherein a first signal is emitted from the first complex bound to the first immobilized capture agent in the first capture zone and a second signal is emitted from the second complex bound to the second immobilized capture agent in the second capture zone, wherein the first signal emitted from the first complex bound to the first immobilized capture agent in the first capture zone decreases as the concentration of the first analyte increases in the fluid sample, and wherein the second signal emitted from the second complex bound to the second immobilized capture agent in the second capture zone increases as the concentration of the second analyte of interest increases in the fluid sample.

17. The lateral flow assay of Claim 13, wherein the first analyte of interest is present in the sample at a concentration about six orders of magnitude greater than the concentration of the second analyte of interest present in the sample,

18. The lateral flow assay of Claim 13, wherein the first analyte of interest is present in the sample at a concentration between 1 and 999 µg/ml and the second analyte of interest is present in the sample at a concentration between 1 and 999 pg/ml.

19. The lateral flow assay of Claim 13, wherein the first analyte of interest is present in the sample at a concentration at least one order of magnitude greater than the concentration of the second analyte of interest present in the sample, the order of magnitude comprising one order of magnitude, two orders of magnitude, three orders of magnitude, four orders of magnitude, five orders of magnitude, six orders of magnitude, seven orders of magnitude, eight orders of magnitude, nine orders of magnitude, or ten orders of magnitude.

20. The lateral flow assay of Claim 13, wherein the first analyte of interest comprises C-reactive protein, CRP, and the second analyte of interest comprises interferon-induced GTP-binding protein, Mx1.

21. The lateral flow assay of Claim 13, wherein the fluid sample is a whole blood sample, a venous blood sample, a capillary blood sample, a serum sample, or a plasma sample optionally, wherein the sample is not diluted prior to applying the sample to the lateral flow assay.

## Patentansprüche

1. Verfahren zum Detektieren eines ersten Analyten von Interesse und eines zweiten Analyten von Interesse, die in einer Probe in unterschiedlichen Konzentrationen vorhanden sind, wobei das Verfahren aufweist:
Bereitstellen eines Lateral-Flow-Assays, der aufweist:
einen ersten Komplex, der an einen Fließweg des Lateral-Flow-Assays gekoppelt ist, wobei der erste Komplex eine Markierung, einen Antikörper oder ein Fragment davon, der spezifisch den ersten Analyten bindet, und den ersten Analyten aufweist,
einen markierten zweiten Antikörper oder ein Fragment davon, der mit dem Fließweg gekoppelt und konfiguriert ist, spezifisch den zweiten Analyten zu binden,
eine erste Einfangzone stromabwärts des ersten Komplexes, wobei die erste Einfangzone ein erstes immobilisiertes Einfangmittel aufweist, das spezifisch für den ersten Analyten ist, und
eine zweite Einfangzone stromabwärts des markierten zweiten Antikörpers oder Fragments davon, die ein zweites immobilisiertes Einfangmittel aufweist, das für den zweiten Analyten spezifisch ist;
Aufbringen der Probe auf den ersten Komplex und den markierten zweiten Antikörper oder ein Fragment davon;
Binden des zweiten Analyten an den markierten zweiten Antikörper oder ein Fragment davon, um einen zweiten Komplex zu bilden;
Fließenlassen der Flüssigkeitsprobe und des ersten Komplexes zur ersten Einfangzone, wobei der erste Analyt in der Flüssigkeitsprobe und der erste Komplex darum konkurrieren, sich an das erste immobilisierte Einfangmittel in der ersten Einfangzone zu binden;
Fließenlassen des zweiten Komplexes im Fließweg zur zweiten Einfangzone und Binden des zweiten Komplexes an das zweite immobilisierte Einfangmittel in der zweiten Einfangzone; und
Detektieren eines ersten Signals vom ersten Komplex, der an das erste immobilisierte Einfangmittel in der ersten Einfangzone gebunden ist, und eines zweiten Signals vom zweiten Komplex, der an das zweite immobilisierte Einfangmittel in der zweiten Einfangzone gebunden ist.

2. Verfahren nach Anspruch 1, wobei der erste Analyt von Interesse in der Probe in einer Konzentration vorhanden ist, die etwa sechs Größenordnungen größer ist als die Konzentration des zweiten Analyten von Interesse in der Probe.

3. Verfahren nach Anspruch 1, wobei der erste Analyt von Interesse in der Probe in einer Konzentration zwischen 1 und 999 µg/ml vorhanden ist und der zweite Analyt von Interesse in der Probe in einer Konzentration zwischen 1 und 999 pg/ml vorhanden ist.

4. Verfahren nach Anspruch 1, wobei der erste Analyt von Interesse in der Probe in einer Konzentration vorhanden ist, die mindestens eine Größenordnung größer ist als die Konzentration des zweiten Analyten von Interesse in der Probe, wobei die Größenordnung eine Größenordnung, zwei Größenordnungen, drei Größenordnungen, vier Größenordnungen, fünf Größenordnungen, sechs Größenordnungen, sieben Größenordnungen, acht Größenordnungen, neun Größenordnungen oder zehn Größenordnungen aufweist.

5. Verfahren nach Anspruch 1, das ferner das Korrelieren des ersten Signals mit einer Konzentration des in der Probe vorhandenen ersten Analyten von Interesse und das Korrelieren des zweiten Signals mit einer Konzentration des zweiten Analyten von Interesse in der Probe aufweist.

6. Verfahren nach Anspruch 1, wobei das erste Signal, das vom ersten Komplex detektiert wird, der an das erste immobilisierte Einfangmittel in der ersten Einfangzone gebunden ist, abnimmt, wenn die Konzentration des ersten Analyten in der Probe zunimmt, und wobei das zweite Signal, das vom zweiten Komplex detektiert wird, der an das zweite immobilisierte Einfangmittel in der zweiten Einfangzone gebunden ist, zunimmt, wenn die Konzentration des zweiten Analyten von Interesse in der Probe zunimmt.

7. Verfahren nach Anspruch 1, das ferner das Detektieren eines dritten Analyten von Interesse in der Probe aufweist, wobei der Lateral-Flow-Assay aufweist:
einen markierten dritten Antikörper oder ein Fragment davon, der mit dem Fließweg gekoppelt und konfiguriert ist, spezifisch den dritten Analyten zu binden; und
eine dritte Einfangzone stromabwärts des markierten dritten Antikörpers oder Fragments davon, die ein drittes immobilisiertes Einfangmittel aufweist, das für den dritten Analyten spezifisch ist, das optional ferner umfasst:
Aufbringen der Probe auf den markierten dritten Antikörper oder ein Fragment davon;
Binden des dritten Analyten an den markierten dritten Antikörper oder ein Fragment davon, um einen dritten Komplex zu bilden;
Fließenlassen des dritten Komplexes im Fließweg zur dritten Einfangzone und Binden des dritten Komplexes an das dritte immobilisierte Einfangmittel in der dritten Einfangzone; und
Detektieren eines dritten Signals vom dritten Komplex, der an das dritte immobilisierte Einfangmittel in der dritten Einfangzone gebunden ist.

8. Verfahren nach Anspruch 7, das ferner das Korrelieren des ersten Signals, des zweiten Signals und des dritten Signals mit einer Konzentration des ersten Analyten, einer Konzentration des zweiten Analyten bzw. einer Konzentration des dritten Analyten in der Probe aufweist.

9. Verfahren nach Anspruch 8, das ferner das Anzeigen eines Krankheitszustands, eines Nicht-Krankheitszustands oder keines Zustands basierend auf den jeweiligen Konzentrationen des ersten Analyten, des zweiten Analyten und des dritten Analyten aufweist, wobei der Krankheitszustand eine virale Infektion oder eine bakterielle Infektion ist und wobei der Nicht-Krankheitszustand eine Entzündung ist.

10. Verfahren nach Anspruch 1, wobei der erste Analyt von Interesse das C-reaktive Protein, CRP, aufweist und der zweite Analyt von Interesse das Interferon-induzierte GTP-bindende Protein, Mx1, aufweist.

11. Verfahren nach Anspruch 7, wobei der dritte Analyt von Interesse Procalcitonin, PCT, aufweist.

12. Verfahren nach Anspruch 1, wobei die Probe eine Vollblutprobe, eine venöse Blutprobe, eine Kapillarblutprobe, eine Serumprobe oder eine Plasmaprobe ist; oder wobei die Probe vor dem Aufbringen der Probe auf den Lateral Flow Assay nicht verdünnt wird.

13. Lateral-Flow-Assay, der konfiguriert ist, einen ersten Analyten von Interesse und einen zweiten Analyten von Interesse, die in einer Flüssigkeitsprobe in unterschiedlichen Konzentrationen vorhanden sind, zu detektieren, wobei der Lateral-Flow-Assay aufweist:
einen ersten Komplex, der an einen Fließweg des Lateral-Flow-Assays gekoppelt ist, wobei der erste Komplex eine Markierung, einen Antikörper oder ein Fragment davon, der spezifisch den ersten Analyten bindet, und den ersten Analyten aufweist;
einen markierten zweiten Antikörper oder ein Fragment davon, der mit dem Fließweg gekoppelt und konfiguriert ist, spezifisch den zweiten Analyten zu binden;
eine erste Einfangzone stromabwärts des ersten Komplexes, wobei die erste Einfangzone ein erstes immobilisiertes Einfangmittel aufweist, das spezifisch für den ersten Analyten ist; und
eine zweite Einfangzone stromabwärts des markierten zweiten Antikörpers oder Fragments davon, die ein zweites immobilisiertes Einfangmittel aufweist, das für den zweiten Analyten spezifisch ist.

14. Lateral-Flow-Assay nach Anspruch 13, wobei der erste Komplex und der markierte zweite Antikörper oder das Fragment davon vom Fließweg des Lateral-Flow-Assays abgekoppelt werden, wenn die Flüssigkeitsprobe auf den ersten Komplex und den markierten zweiten Antikörper oder das Fragment davon aufgebracht wird, wobei sich optional der markierte zweite Antikörper oder das Fragment davon an den zweiten Analyten bindet, um einen zweiten Komplex zu bilden, wenn die Flüssigkeitsprobe auf den ersten Komplex und den markierten zweiten Antikörper oder das Fragment davon aufgebracht wird.

15. Lateral-Flow-Assay nach Anspruch 14, wobei nachdem die Flüssigkeitsprobe aufgebracht ist, sich das erste immobilisierte Einfangmittel in der ersten Einfangzone konkurrierend an den ersten Komplex und den ersten Analyten bindet, die in der Flüssigkeitsprobe in die erste Einfangzone geflossen sind, wobei optional nachdem die Flüssigkeitsprobe aufgebracht ist, sich das zweite immobilisierte Einfangmittel in der zweiten Einfangzone an den zweiten Komplex bindet, der in der Flüssigkeitsprobe in die zweite Einfangzone geflossen ist.

16. Lateral-Flow-Assay nach Anspruch 15, wobei ein erstes Signal vom ersten Komplex emittiert wird, der an das erste immobilisierte Einfangmittel in der ersten Einfangzone gebunden ist, und ein zweites Signal von dem zweiten Komplex emittiert wird, der an das zweite immobilisierte Einfangmittel in der zweiten Einfangzone gebunden ist, wobei das erste Signal, das von dem ersten Komplex emittiert wird, der an das erste immobilisierte Einfangmittel in der ersten Einfangzone gebunden ist, abnimmt, wenn die Konzentration des ersten Analyten in der Flüssigkeitsprobe zunimmt, und wobei das zweite Signal, das von dem zweiten Komplex emittiert wird, der an das zweite immobilisierte Einfangmittel in der zweiten Einfangzone gebunden ist, zunimmt, wenn die Konzentration des zweiten Analyten von Interesse in der Flüssigkeitsprobe zunimmt.

17. Lateral-Flow-Assay nach Anspruch 13, wobei der erste Analyt von Interesse in der Probe in einer Konzentration vorhanden ist, die etwa sechs Größenordnungen größer ist als die Konzentration des zweiten Analyten von Interesse in der Probe.

18. Lateral-Flow-Assay nach Anspruch 13, wobei der erste Analyt von Interesse in der Probe in einer Konzentration zwischen 1 und 999 µg/ml vorhanden ist und der zweite Analyt von Interesse in der Probe in einer Konzentration zwischen 1 und 999 pg/ml vorhanden ist.

19. Lateral-Flow-Assay nach Anspruch 13, wobei der erste Analyt von Interesse in der Probe in einer Konzentration vorhanden ist, die mindestens eine Größenordnung größer ist als die Konzentration des zweiten Analyten von Interesse in der Probe, wobei die Größenordnung eine Größenordnung, zwei Größenordnungen, drei Größenordnungen, vier Größenordnungen, fünf Größenordnungen, sechs Größenordnungen, sieben Größenordnungen, acht Größenordnungen, neun Größenordnungen oder zehn Größenordnungen aufweist.

20. Lateral-Flow-Assay nach Anspruch 13, wobei der erste Analyt von Interesse das C-reaktive Protein, CRP, aufweist und der zweite Analyt von Interesse das Interferon-induzierte GTP-bindende Protein, Mx1, aufweist.

21. Lateral-Flow-Assay nach Anspruch 13, wobei die Flüssigkeitsprobe eine Vollblutprobe, eine venöse Blutprobe, eine Kapillarblutprobe, eine Serumprobe oder eine Plasmaprobe ist, wobei die Probe vor dem Aufbringen der Probe auf den Lateral-Flow-Assay nicht verdünnt wird.

## Revendications

1. Procédé de détection d'un premier analyte d'intérêt et d'un deuxième analyte d'intérêt présents dans un échantillon à des concentrations différentes, ledit procédé comprenant :
la préparation d'un dosage à écoulement latéral, comprenant
un premier complexe relié à une voie d'écoulement du dosage à écoulement latéral, ledit premier complexe comprenant un marqueur, un anticorps ou un fragment de celui-ci liant spécifiquement le premier analyte, et le premier analyte,
un deuxième anticorps marqué ou un fragment de celui-ci relié à la voie d'écoulement et prévu pour lier spécifiquement le deuxième analyte,
une première zone de capture en aval du premier complexe, ladite première zone de capture comprenant un premier agent de capture immobilisé spécifique au premier analyte, et
une deuxième zone de capture en aval du deuxième anticorps marqué ou de son fragment et comprenant un deuxième agent de capture immobilisé spécifique au deuxième analyte ;
l'application de l'échantillon au premier complexe et au deuxième anticorps marqué ou à son fragment ;
la liaison du deuxième analyte au deuxième anticorps marqué ou à son fragment pour former un deuxième complexe ;
l'écoulement de l'échantillon de fluide et du premier complexe dans la première zone de capture, le premier analyte dans l'échantillon de fluide et le premier complexe étant en compétition pour lier le premier agent de capture immobilisé dans la première zone de capture ;
l'écoulement du deuxième complexe dans la voie d' écoulement jusqu' à la deuxième zone de capture et la liaison du deuxième complexe au deuxième agent de capture immobilisé dans la deuxième zone de capture ; et
la détection d'un premier signal provenant du premier complexe lié au premier agent de capture immobilisé dans la première zone de capture et d'un deuxième signal provenant du deuxième complexe lié au deuxième agent de capture immobilisé dans la deuxième zone de capture.

2. Procédé selon la revendication 1, où le premier analyte d'intérêt est présent dans l'échantillon à une concentration supérieure d'environ six ordres de grandeur à la concentration du deuxième analyte d'intérêt présent dans l'échantillon.

3. Procédé selon la revendication 1, où le premier analyte d'intérêt est présent dans l'échantillon à une concentration comprise entre 1 et 999 µg/ml et le deuxième analyte d'intérêt est présent dans l'échantillon à une concentration comprise entre 1 et 999 pg/ml.

4. Procédé selon la revendication 1, où le premier analyte d'intérêt est présent dans l'échantillon à une concentration supérieure d'au moins un ordre de grandeur à la concentration du deuxième analyte d'intérêt présent dans l'échantillon, ledit ordre de grandeur comprenant un ordre de grandeur, deux ordres de grandeur, trois ordres de grandeur, quatre ordres de grandeur, cinq ordres de grandeur, six ordres de grandeur, sept ordres de grandeur, huit ordres de grandeur, neuf ordres de grandeur ou dix ordres de grandeur.

5. Procédé selon la revendication 1, comprenant en outre la corrélation du premier signal à une concentration du premier analyte d'intérêt présent dans l'échantillon et la corrélation du deuxième signal à une concentration du deuxième analyte d'intérêt dans l'échantillon.

6. Procédé selon la revendication 1, où le premier signal détecté à partir du premier complexe lié au premier agent de capture immobilisé dans la première zone de capture décroît lorsque la concentration du premier analyte augmente dans l'échantillon, et où le deuxième signal détecté à partir du deuxième complexe lié au deuxième agent de capture immobilisé dans la deuxième zone de capture croît lorsque la concentration du deuxième analyte d'intérêt augmente dans l'échantillon.

7. Procédé selon la revendication 1, comprenant en outre la détection d'un troisième analyte d'intérêt dans l'échantillon, où le dosage à écoulement latéral comprend :
un troisième anticorps marqué ou un fragment de celui-ci relié à la voie d'écoulement et prévu pour lier spécifiquement le troisième analyte ; et
une troisième zone de capture en aval du troisième anticorps marqué ou de son fragment et comprenant un troisième agent de capture immobilisé spécifique au troisième analyte, facultativement comprenant en outre :
l'application de l'échantillon au troisième anticorps marqué ou à son fragment ;
la liaison du troisième analyte au troisième anticorps marqué ou à son fragment pour former un troisième complexe ;
l'écoulement du troisième complexe dans la voie d'écoulement jusqu'à la troisième zone de capture et la liaison du troisième complexe au troisième agent de capture immobilisé dans la troisième zone de capture ; et
la détection d'un troisième signal provenant du troisième complexe lié au troisième agent de capture immobilisé dans la troisième zone de capture.

8. Procédé selon la revendication 7, comprenant en outre la corrélation du premier signal, du deuxième signal et du troisième signal avec une concentration du premier analyte, une concentration du deuxième analyte et une concentration du troisième analyte dans l'échantillon, respectivement.

9. Procédé selon la revendication 8, comprenant en outre l'indication d'un état pathologique, d'un état non pathologique ou de l'absence d'état sur la base des concentrations respectives du premier analyte, du deuxième analyte et du troisième analyte, où l'état pathologique est une infection virale ou une infection bactérienne, et où l'état non pathologique est une inflammation.

10. Procédé selon la revendication 1, où le premier analyte d'intérêt comprend une protéine C réactive, CRP, et le deuxième analyte d'intérêt comprend une protéine de liaison GTP induite par interféron, Mx1.

11. Procédé selon la revendication 7, où le troisième analyte d'intérêt comprend une procalcitonine, PCT.

12. Procédé selon la revendication 1, où l'échantillon est un échantillon de sang total, un échantillon de sang veineux, un échantillon de sang capillaire, un échantillon de sérum, ou un échantillon de plasma ; ou où l'échantillon n'est pas dilué avant application de l'échantillon au dosage à écoulement latéral.

13. Dosage à écoulement latéral prévu pour détecter un premier analyte d'intérêt et un deuxième analyte d'intérêt présents dans un échantillon de fluide à des concentrations différentes, ledit dosage à écoulement latéral comprenant :
un premier complexe relié à une voie d'écoulement du dosage à écoulement latéral, ledit premier complexe comprenant un marqueur, un anticorps ou un fragment de celui-ci liant spécifiquement le premier analyte, et le premier analyte,
un deuxième anticorps marqué ou un fragment de celui-ci relié à la voie d'écoulement et prévu pour lier spécifiquement le deuxième analyte,
une première zone de capture en aval du premier complexe, ladite première zone de capture comprenant un premier agent de capture immobilisé spécifique au premier analyte ; et
une deuxième zone de capture en aval du deuxième anticorps marqué ou de son fragment et comprenant un deuxième agent de capture immobilisé spécifique au deuxième analyte.

14. Dosage à écoulement latéral selon la revendication 13, où le premier complexe et le deuxième anticorps marqué ou son fragment se dissocient de la voie d'écoulement du dosage à écoulement latéral lorsque l'échantillon de fluide est appliqué au premier complexe et au deuxième anticorps marqué ou à son fragment, facultativement où le deuxième anticorps marqué ou son fragment lient le deuxième analyte pour former un deuxième complexe lorsque l'échantillon de fluide est appliqué au premier complexe et au deuxième anticorps marqué ou à son fragment.

15. Dosage à écoulement latéral selon la revendication 14, où, après application de l'échantillon de fluide, le premier agent de capture immobilisé dans la première zone de capture lie de manière compétitive le premier complexe et le premier analyte qui se sont écoulés dans l'échantillon de fluide vers la première zone de capture, facultativement où, après application de l'échantillon de fluide, le deuxième agent de capture immobilisé dans la deuxième zone de capture lie le deuxième complexe qui s'est écoulé dans l'échantillon de fluide vers la deuxième zone de capture.

16. Dosage à écoulement latéral selon la revendication 15, où un premier signal est émis par le premier complexe lié au premier agent de capture immobilisé dans la première zone de capture et un deuxième signal est émis par le deuxième complexe lié au deuxième agent de capture immobilisé dans la deuxième zone de capture, où le premier signal émis par le premier complexe lié au premier agent de capture immobilisé dans la première zone de capture décroît lorsque la concentration du premier analyte augmente dans l'échantillon de fluide, et le deuxième signal émis par le deuxième complexe lié au deuxième agent de capture immobilisé dans la deuxième zone de capture croît lorsque la concentration du deuxième analyte d'intérêt augmente dans l'échantillon de fluide.

17. Dosage à écoulement latéral selon la revendication 13, où le premier analyte d'intérêt est présent dans l'échantillon à une concentration d'environ six ordres de grandeur supérieure à la concentration du deuxième analyte d'intérêt présent dans l'échantillon.

18. Dosage à écoulement latéral selon la revendication 13, où le premier analyte d'intérêt est présent dans l'échantillon à une concentration comprise entre 1 et 999 µg/ml et le deuxième analyte d'intérêt est présent dans l'échantillon à une concentration comprise entre 1 et 999 pg/ml.

19. Dosage à écoulement latéral selon la revendication 13, où le premier analyte d'intérêt est présent dans l'échantillon à une concentration supérieure d'au moins un ordre de grandeur à la concentration du deuxième analyte d'intérêt présent dans l'échantillon, ledit ordre de grandeur comprenant un ordre de grandeur, deux ordres de grandeur, trois ordres de grandeur, quatre ordres de grandeur, cinq ordres de grandeur, six ordres de grandeur, sept ordres de grandeur, huit ordres de grandeur, neuf ordres de grandeur ou dix ordres de grandeur.

20. Dosage à écoulement latéral selon la revendication 13, où le premier analyte d'intérêt comprend une protéine C réactive, CRP, et le deuxième analyte d'intérêt comprend une protéine de liaison GTP induite par interféron, Mx1.

21. Dosage à écoulement latéral selon la revendication 13, où l'échantillon de fluide est un échantillon de sang total, un échantillon de sang veineux, un échantillon de sang capillaire, un échantillon de sérum, ou un échantillon de plasma, facultativement où l'échantillon n'est pas dilué avant application de l'échantillon au dosage à écoulement latéral.
